# EUROPÄISCHE PATENTSCHRIFT

(11) **EP 2 892 876 B1**
(45) Veröffentlichungstag und Bekanntmachung des Hinweises auf die Patenterteilung: **07.11.2018**
(21) Anmeldenummer: 13748265.9
(22) Anmeldetag: 06.08.2013
(51) Int. Cl.: C07C 211/61, C07F 7/08, C07D 307/91, C09K 11/06, H05B 33/14, C09K 19/30, C07C 255/58, C07B 59/00, C07F 9/6568, C07C 209/68, C07C 217/92, C07C 229/52, H05B 33/10, H01L 51/50, H01L 51/00, C07D 471/04, C07D 487/04, C07D 493/04, C07D 495/04, C07D 519/00

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 04.09.2012 EP 12006239
(43) Veröffentlichungstag der Anmeldung: 15.07.2015
(73) Patentinhaber: Merck Patent GmbH, 64293 Darmstadt (DE)
(72) Erfinder: HEIL, Holger, 60389 Frankfurt am Main (DE); RODRIGUEZ, Lara-Isabel, 64293 Darmstadt (DE); ECKES, Fabrice, 64293 Darmstadt (DE); KAUFHOLD, Oliver, 64297 Darmstadt (DE); GERHARD, Anja, 63329 Egelsbach (DE); RIEDMUELLER, Stefan, 60431 Frankfurt am Main (DE)
(86) Internationale Anmeldenummer: PCT/EP2013/002343
(87) Internationale Veröffentlichungsnummer: WO 2014/037077

(56) Entgegenhaltungen:
- WO-A1-2008/006449
- WO-A1-2010/012328
- WO-A1-2011/136484
- US-A1- 2009 066 225
- US-A1- 2012 012 832

## Beschreibung

Die vorliegende Erfindung betrifft eine Verbindung, die Verwendung der Verbindung in einer elektronischen Vorrichtung, sowie eine elektronische Vorrichtung enthaltend die Verbindung.

Es ist aktuell von Interesse, Verbindungen zu entwickeln, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung allgemein elektronische Vorrichtungen verstanden, welche organische Materialien enthalten. Insbesondere werden darunter verstanden organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs).

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen.

Von großer Bedeutung ist in diesem Zusammenhang die Wahl der Verbindung, welche als Dotand bzw. als emittierende Verbindung in der OLED eingesetzt wird.

Im Stand der Technik sind hierzu eine Vielzahl an Verbindungen bekannt, insbesondere Arylamine mit einer oder mehreren kondensierten Arylgruppen.

Beispielhaft sind hier die in WO 2008/006449 und WO 2010/012328 offenbarten Verbindungen zu nennen, welche auf einem Indenofluoren-Gerüst beruhen, bei dem eine der Phenylgruppen zu einer größeren Arylgruppe erweitert ist, beispielsweise zu einer Naphthyl- oder einer Pyrenylgruppe. Die Verbindungen weisen zusätzlich eine Aminogruppe auf, welche eine wahlweise substituierte Diphenylaminogruppe darstellt. In US 2012/012832, WO 2011/136484 und US 2009/066225 werden Verbindungen offenbart, die auf einem Indenofluoren-Gerüst beruhen, und die mindestens eine Aminogruppe enthalten.

Die in den oben genannten Anmeldungen offenbarten Verbindungen sind zwar wertvolle funktionelle Verbindungen, sie sind jedoch noch nicht optimal für die Verwendung als tiefblaue Emitter in OLEDs geeignet. Insbesondere gibt es aufgrund der immer weiter steigenden Anforderungen kontinuierlichen Verbesserungsbedarf in Bezug auf zentrale Device-Parameter wie Leistungseffizienz und Lebensdauer.

Die im Folgenden definierte neue Verbindung löst diese technische Aufgabe.

Gegenstand der vorliegenden Erfindung ist eine Verbindung der Formel (I), (II) oder (III) 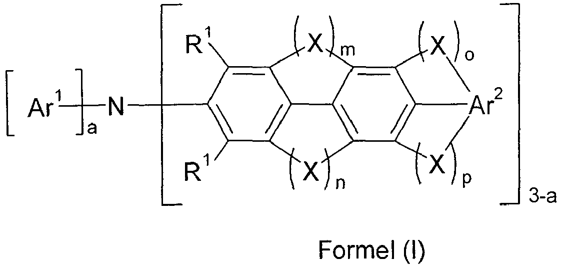 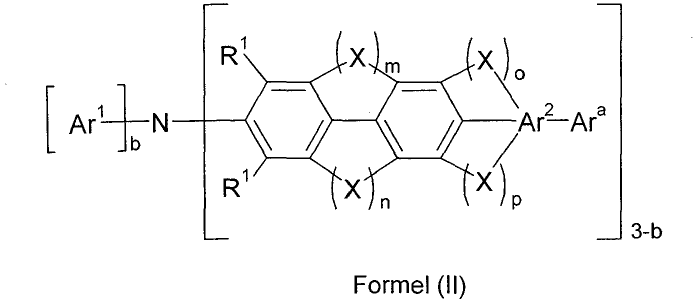 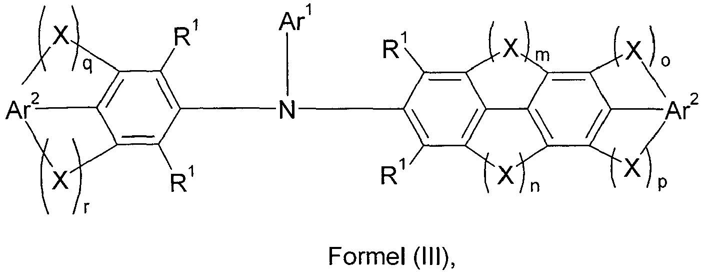 wobei gilt:
- Ar¹: ist bei jedem Auftreten gleich oder verschieden gewählt aus einem aromatischen oder heteroaromatischen Ringsystem mit 5 bis 30 aromatischen Ringatomen, welches mit einem oder mehreren Resten R¹ substituiert sein kann;
- Ar²: ist bei jedem Auftreten gleich oder verschieden eine Aryl- oder Heteroarylgruppe mit 10 bis 30 aromatischen Ringatomen, welche mit einem oder mehreren Resten R¹ substituiert sein kann;
- Ar^{a}: ist bei jedem Auftreten gleich oder verschieden eine Aryl- oder Heteroarylgruppe mit 5 bis 30 aromatischen Ringatomen, welche mit einem oder mehreren Resten R¹ substituiert sein kann;
- R¹: ist bei jedem Auftreten gleich oder verschieden H, D, F, Cl, Br, I, C(=O)R², CN, Si(R²)₃, N(R²)₂, NO₂, P(=O)(R²)₂, S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, oder eine Aryloxy- oder Heteroaryloxygruppe mit 5 bis 30 aromatischen Ringatomen, die durch einen oder mehrere Reste R² substituiert sein kann, wobei zwei Reste R¹, welche an dieselbe Gruppe X gebunden sind, miteinander einen Ring bilden können;
- R²: ist bei jedem Auftreten gleich oder verschieden H, D, F, Cl, Br, I, C(=O)R³, CN, Si(R³)₃, N(R³)₂, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
- R³: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
- X: ist bei jedem Auftreten gleich oder verschieden C(R¹)₂ oder Si(R¹)₂;
- a: ist gleich 0 oder 1;
- b: ist gleich 0, 1 oder 2;
- m, n, o, p, q und r: sind bei jedem Auftreten gleich oder verschieden 0 oder 1; wobei im Fall, dass sie 0 sind, an den betreffenden Positionen, an die die entsprechende Gruppe X bindet, stattdessen eine Gruppe R¹ gebunden ist;
wobei die Summe von m und n gleich 1 ist, und die Summe von o und p gleich 1 ist, und die Summe von q und r gleich 1 oder 2 ist.

Die Verbindung eignet sich hervorragend zur Verwendung in OLEDs. Insbesondere eignet sie sich zur Verwendung als tiefblau emittierende Emitterverbindung. Insbesondere führt sie bei Verwendung in OLEDs zu einer Verbesserung in Bezug auf die Lebensdauer der Vorrichtungen und/oder die Leistungseffizienz.

Im Folgenden sind allgemeine Begriffsdefinitionen aufgeführt, die im Rahmen der vorliegenden Anmeldung gelten.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

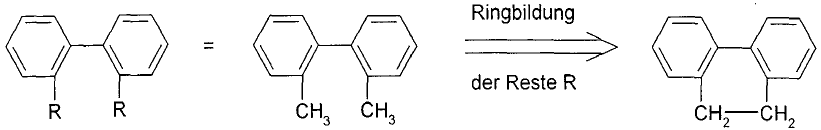

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

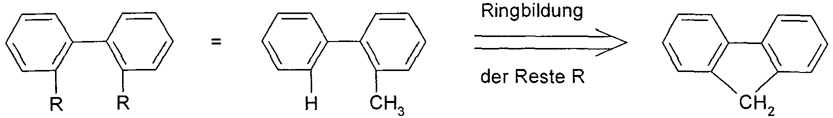

Ar¹ ist bevorzugt bei jedem Auftreten gleich oder verschieden gewählt aus einem aromatischen oder heteroaromatischen Ringsystem mit 5 bis 24 aromatischen Ringatomen, das mit einem oder mehreren Resten R¹ substituiert sein kann. Besonders bevorzugt ist Ar¹ ein aromatisches oder heteroaromatisches Ringsystem mit 6 bis 18 aromatischen Ringatomen, das mit einem oder mehreren Resten R¹ substituiert sein kann.

Weiterhin bevorzugt ist Ar¹ eine Aryl- oder Heteroarylgruppe mit 6 bis 16 aromatischen Ringatomen, die mit einem oder mehreren Resten R¹ substituiert ist.

Ganz besonders bevorzugt ist Ar¹ bei jedem Auftreten gleich oder verschieden gewählt aus Phenyl, Naphthyl, Anthracenyl, Phenanthrenyl, Pyrenyl, Triphenylenyl, Chrysenyl, Biphenyl, Terphenyl, Fluorenyl, Spirobifluorenyl, Carbazolyl, Dibenzofuranyl, Dibenzothiophenyl oder Silafluorenyl, das jeweils mit einem oder mehreren Resten R¹ substituiert sein kann.

Weiterhin bevorzugt ist Ar¹ bei jedem Auftreten in einer Formel gleich gewählt.

Ar² ist bevorzugt bei jedem Auftreten gleich oder verschieden gewählt aus einer Arylgruppe mit 10 bis 22 aromatischen Ringatomen, welche mit einem oder mehreren Resten R¹ substituiert sein kann, besonders bevorzugt aus einer Arylgruppe mit 10 bis 18 aromatischen Ringatomen, welche mit einem oder mehreren Resten R¹ substituiert sein kann.

Ganz besonders bevorzugt ist Ar² gewählt aus den folgenden Gruppen der Formeln (Ar²-a) bis (Ar²-h):

| | |
|---|---|
| 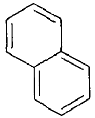 | 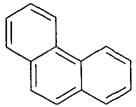 |
| Formel (Ar²-a) | Formel (Ar²-b) |
| 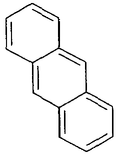 | 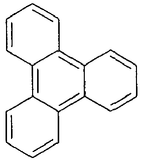 |
| Formel (Ar²-c) | Formel (Ar²-d) |
| 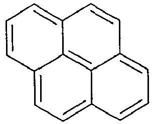 | 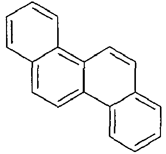 |
| Formel (Ar²-e) | Formel (Ar²-f) |
| 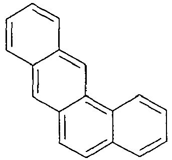 | 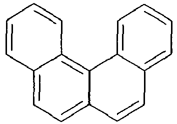 |
| Formel (Ar²-g) | Formel (Ar²-h) |

wobei die Anbindungspositionen an den Rest der Formel an beliebigen Positionen vorliegen können und wobei die Gruppen mit einem oder mehreren Resten R¹ substituiert sein können. Bevorzugt wird dabei durch die Brücke X mit Ar² und der Phenylgruppe ein Fünf- oder ein Sechsring aufgespannt, besonders bevorzugt ein Fünfring.

Weiterhin bevorzugt ist Ar² bei jedem Auftreten gleich gewählt.

Für die Verbindungen der Formel (III) ist es bevorzugt, dass die Gruppe Ar² mit einer oder mehreren Gruppen R¹ substituiert ist, die gewählt sind aus einem aromatischen oder heteroaromatischen Ringsystem mit 5 bis 30 aromatischen Ringatomen, das mit einem oder mehreren Resten R² substituiert sein kann. Besonders bevorzugt ist die in Formel (III) links stehende Gruppe Ar² mit einer oder mehreren Gruppen R¹ substituiert, die gewählt sind aus einem aromatischen oder heteroaromatischen Ringsystem mit 5 bis 30 aromatischen Ringatomen, das mit einem oder mehreren Resten R² substituiert sein kann. Besonders bevorzugt sind als Substituenten R¹ der Gruppe Ar² , insbesondere der linken Gruppe Ar² in Formel (III), Aryl- oder Heteroarylgruppen mit 6 bis 16 aromatischen Ringatomen, die mit einem oder mehreren Resten R² substituiert sein können.

Die Gruppe Ar^{a} ist bevorzugt bei jedem Auftreten gleich oder verschieden eine Aryl- oder Heteroarylgruppe mit 5 bis 18 aromatischen Ringatomen, besonders bevorzugt 5 bis 14 aromatischen Ringatomen, ganz besonders bevorzugt 5 bis 10 aromatischen Ringatomen, wobei Ar^{a} mit einem oder mehreren Resten R¹ substituiert sein kann.

Weiterhin ist es bevorzugt, dass Ar^{a} eine Arylgruppe mit 6 bis 18 aromatischen Ringatomen, besonders bevorzugt 6 bis 14 aromatischen Ringatomen und ganz besonders bevorzugt 6 bis 10 aromatischen Ringatomen darstellt, wobei Ar^{a} mit einem oder mehreren Resten R¹ substituiert sein kann.

Weiterhin bevorzugt ist Ar^{a} bei jedem Auftreten gleich gewählt.

Die Gruppe X ist bevorzugt bei jedem Auftreten gleich oder verschieden C(R¹)2.

Bevorzugt ist R¹ bei jedem Auftreten gleich oder verschieden H, D, F, CN, Si(R²)₃, N(R²)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R² substituiert sein können und wobei in den oben genannten Gruppen eine oder mehrere CH₂-Gruppen durch -C≡C-, -R²C=CR²-, Si(R²)₂, C=O, C=NR², -NR²-, -O-, -S-, -C(=O)O- oder -C(=O)NR²- ersetzt sein können, oder ein aromatisches oder heteroaromatisches Ringsystem mit 5 bis 20 aromatischen Ringatomen, das jeweils mit einem oder mehreren Resten R² substituiert sein kann, wobei zwei Reste R¹, welche an dieselbe Gruppe X gebunden sind, miteinander einen Ring bilden können.

Besonders bevorzugt ist R¹ bei jedem Auftreten gleich oder verschieden gewählt aus H, D, F, CN, Si(R²)₃, einer geradkettigen Alkylgruppe mit 1 bis 8 C-Atomen oder einer verzweigten oder cyclischen Alkylgruppe mit 3 bis 8 C-Atomen, wobei die Alkylgruppen jeweils mit einem oder mehreren Resten R² substituiert sein können und wobei in den Alkylgruppen eine oder mehrere CH₂-Gruppen durch -C≡C-, -R²C=CR²-, Si(R²)₂, C=O oder -O- ersetzt sein können, oder einer Aryl- oder Heteroarylgruppe mit 6 bis 16 aromatischen Ringatomen, die jeweils mit einem oder mehreren Resten R² substituiert sein kann.

Bevorzugt ist R¹, welches an Gruppen X, insbesondere Gruppen X, die C(R¹)₂ darstellen, gebunden ist, bei jedem Auftreten gleich oder verschieden gewählt aus Alkylgruppen mit 1 bis 10 C-Atomen, welche mit einem oder mehreren Resten R² substituiert sein können, und aromatischen oder heteroaromatischen Ringsystemen mit 6 bis 12 aromatischen Ringatomen, welche mit einem oder mehreren Resten R² substituiert sein können. Dabei können zwei Reste R¹, welche an dieselbe Gruppe X, insbesondere bei Gruppen X, die C(R¹)₂ darstellen, gebunden sind, miteinander einen Ring bilden.

Bevorzugt ist R² bei jedem Auftreten gleich oder verschieden H, D, F, CN, Si(R³)₃, N(R³)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, - R³C=CR³-, Si(R³)₂, C=O, C=NR³, -NR³-, -O-, -S-, -C(=O)O- oder-C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann.

Besonders bevorzugt ist R² bei jedem Auftreten gleich oder verschieden gewählt aus H, D, F, CN, einer geradkettigen Alkylgruppe mit 1 bis 8 C-Atomen oder einer verzweigten oder cyclischen Alkylgruppe mit 3 bis 8 C-Atomen, wobei die Alkylgruppen jeweils mit einem oder mehreren Resten R² substituiert sein können und wobei in den Alkylgruppen eine oder mehrere CH₂-Gruppen durch -C≡C-, -R³C=CR³-, Si(R³)₂, C=O oder -O-ersetzt sein können, oder einer Aryl- oder Heteroarylgruppe mit 6 bis 16 aromatischen Ringatomen, die jeweils mit einem oder mehreren Resten R³ substituiert sein kann.

Weiterhin ist es bevorzugt, dass a gleich 1 ist.

Weiterhin ist es bevorzugt, dass b gleich 2 ist.

Weiterhin ist es bevorzugt, dass die Summe aus q und r gleich 1 ist.

Weiterhin bevorzugt ist es, dass m und p gleich 1 sind und n und o gleich 0.

Weiterhin bevorzugt ist es, dass n und o gleich 1 sind und m und p gleich 0.

Bevorzugte Ausführungsformen von Formel (I) entsprechen den folgenden Formeln (I-1) bis (I-12):

| |
|---|
| 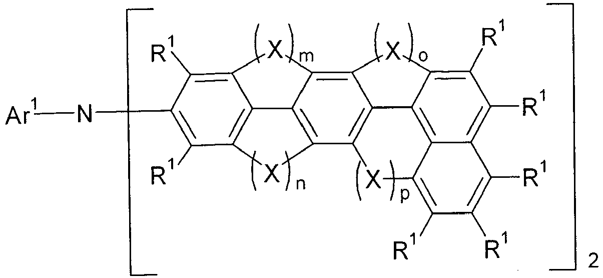 |
| Formel (I-1) |
| 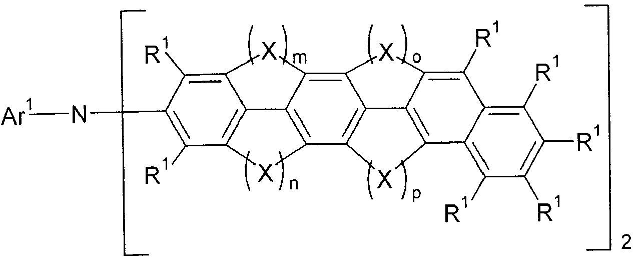 |
| Formel (I-2) |
| 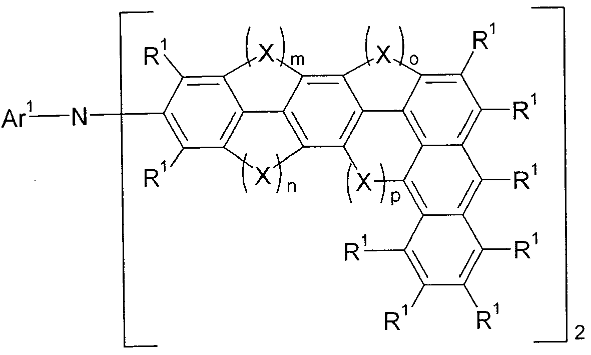 |
| Formel (I-3) |
| 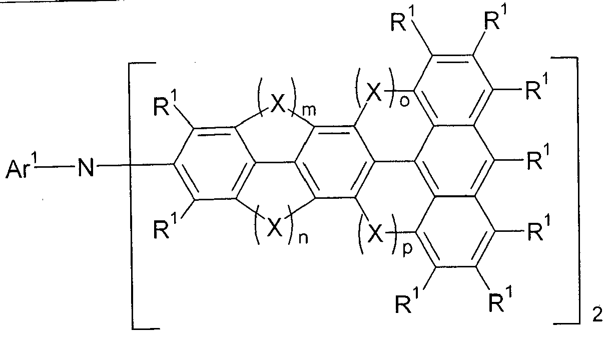 |
| Formel (I-4) |
| 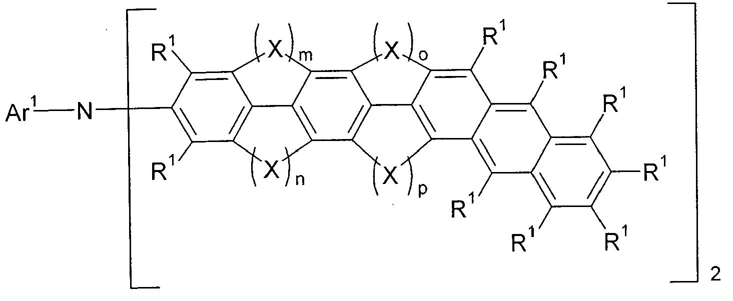 |
| Formel I-5 |
| 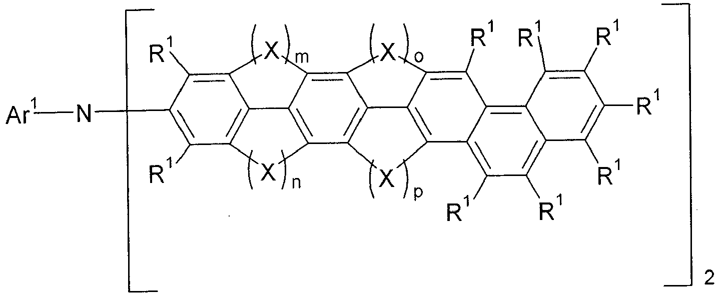 |
| Formel I-6 |
| 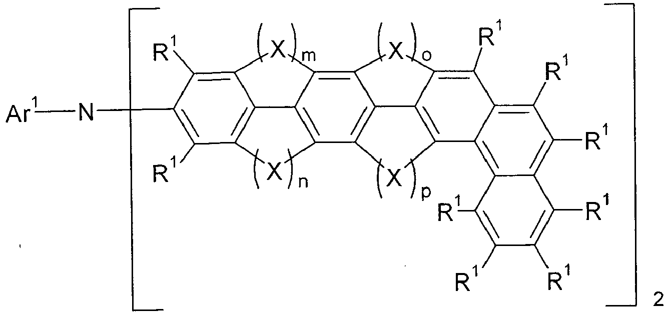 |
| Formel (I-7) |
| 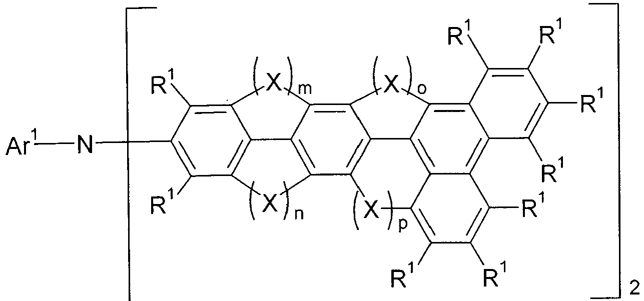 |
| Formel (I-8) |
| 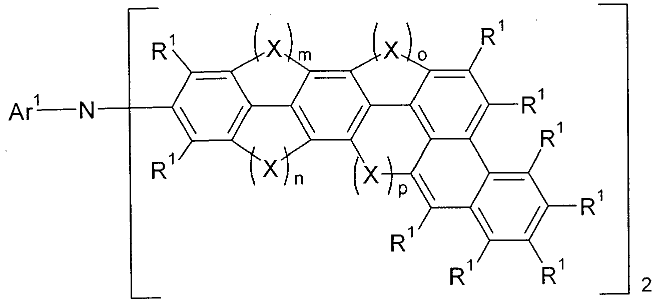 |
| Formel (I-9) |
| 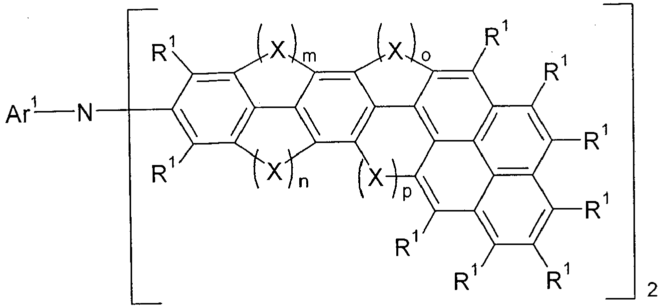 |
| Formel (I-10) |
| 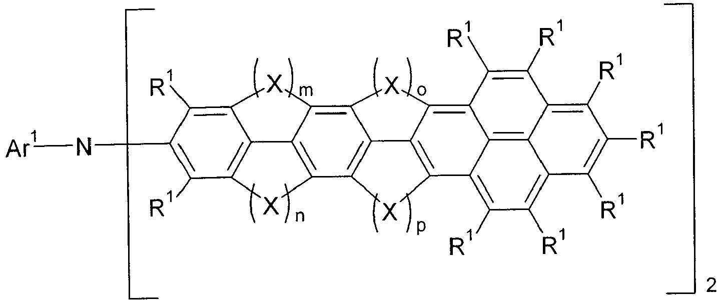 |
| Formel (I-11) |
| 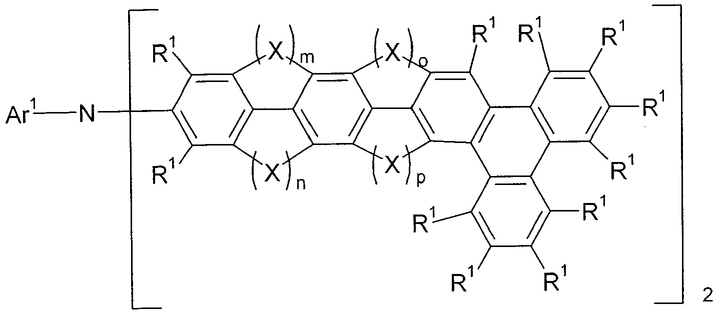 |
| Formel (I-12) |

wobei die auftretenden Symbole und Indices wie oben definiert sind. Es gelten bevorzugt die oben angegebenen bevorzugten Ausführungsformen der Symbole und Indices.

Es ist für die obenstehenden Formeln bevorzugt, wenn m und p gleich 1 ist und n und o gleich 0 ist. Alternativ ist es für obenstehende Formeln bevorzugt, wenn n und o gleich 1 ist und m und p gleich 0 ist.

Bevorzugte Ausführungsformen von Formel (II) entsprechen den folgenden Formeln (II-1) bis (II-12):

| |
|---|
| 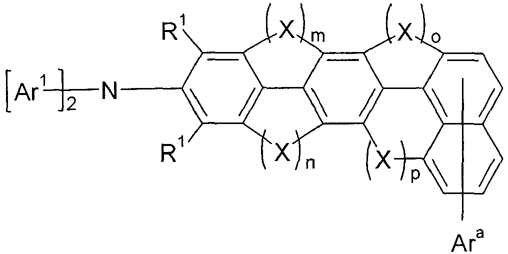 |
| Formel (II-1) |
| 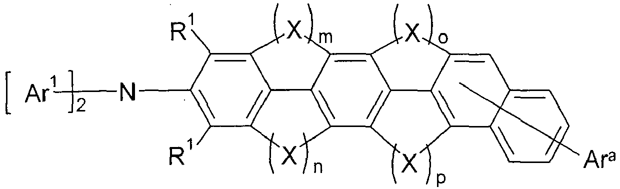 |
| Formel (II-2) |
| 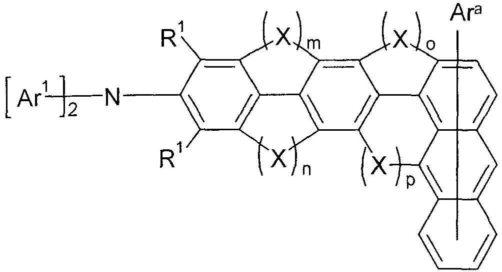 |
| Formel (II-3) |
| 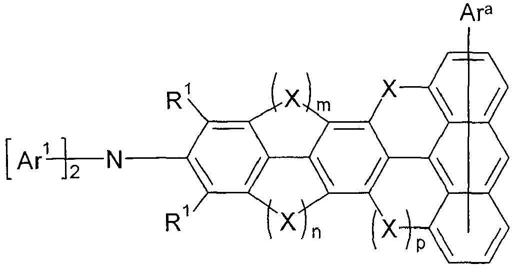 |
| Formel (II-4) |
| 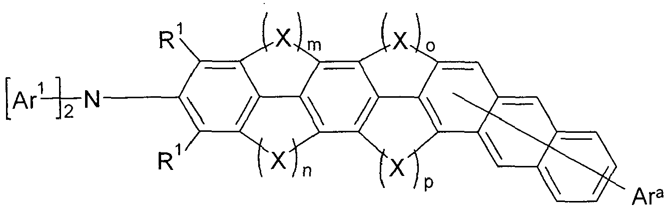 |
| Formel (II-5) |
| 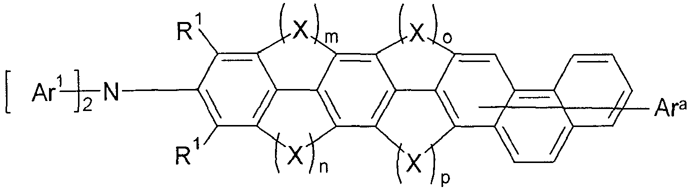 |
| Formel(II-6) |
| 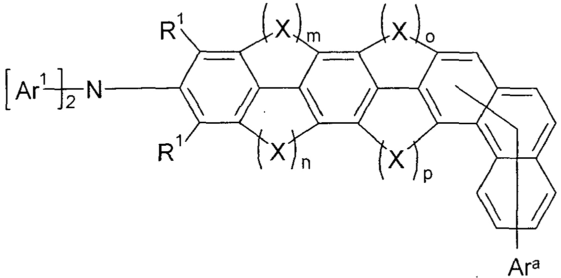 |
| Formel (II-7) |
| 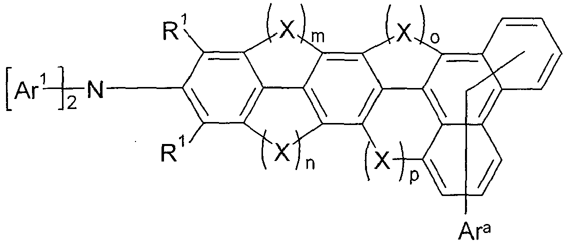 |
| Formel (II-8) |
| 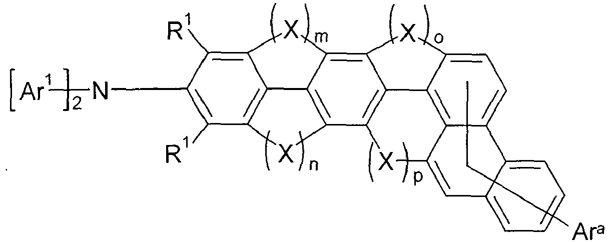 |
| Formel (II-9) |
| 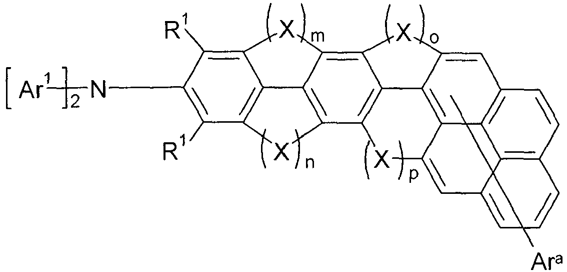 |
| Formel (II-10) |
| 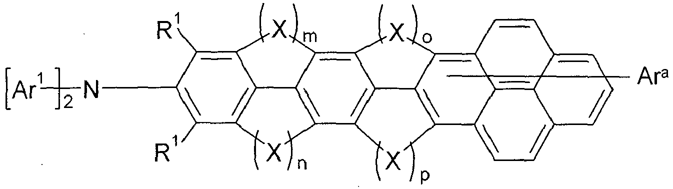 |
| Formel (II-11) |
| 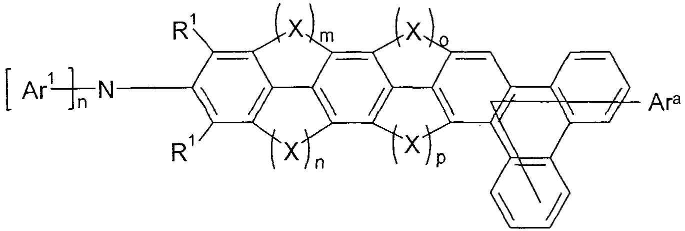 |
| Formel (II-12) |

wobei die auftretenden Symbole und Indices wie oben definiert sind und wobei Ar^{a} an jeder beliebigen freien Position an der endständigen Arylgruppe binden kann und wobei die endständige Arylgruppe an den anderen freien Positionen jeweils mit Gruppen R¹ substituiert sein kann.

Es gelten bevorzugt die oben angegebenen bevorzugten Ausführungsformen der Symbole und Indices, insbesondere die für Ar^{a}.

Es ist für die obenstehenden Formeln bevorzugt, wenn m und p gleich 1 ist und n und o gleich 0 ist. Alternativ ist es für obenstehende Formeln bevorzugt, wenn n und o gleich 1 ist und m und p gleich 0 ist.

Bevorzugte Ausführungsformen von Formel (III) entsprechen den folgenden Formeln (III-1) bis (III-4):

| |
|---|
| 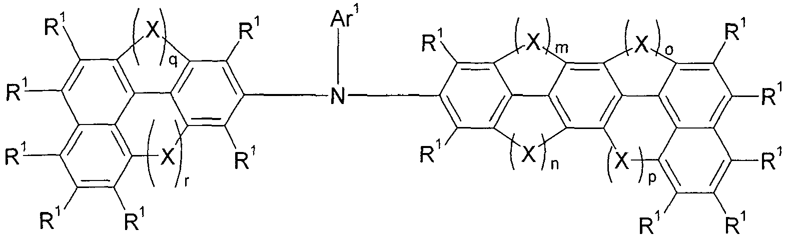 |
| Formel (III-1) |
| 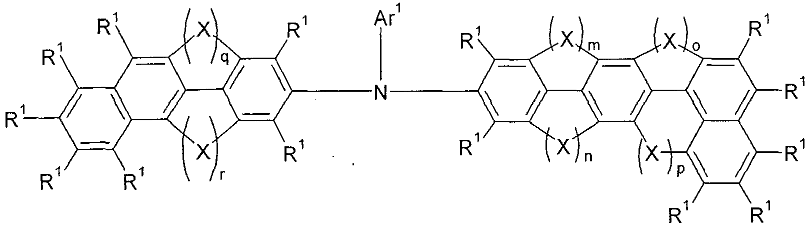 |
| Formel (III-2) |
| 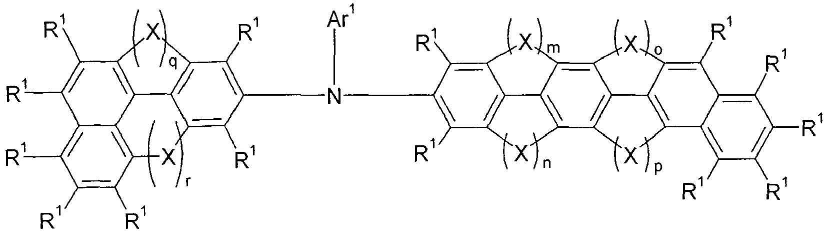 |
| Formel (III-3) |
| 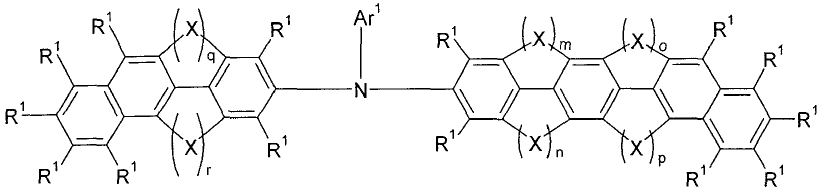 |
| Formel (III-4), |

wobei die auftretenden Symbole und Indices wie oben definiert sind. Es gelten bevorzugt die oben angegebenen bevorzugten Ausführungsformen der Symbole und Indices.

Besonders bevorzugt ist unter den abgebildeten Formeln (III-1) bis (III-4) Formel (III-1).

Es ist für die obenstehenden Formeln bevorzugt, wenn m und p gleich 1 ist und n und o gleich 0 ist. Alternativ ist es für obenstehende Formeln bevorzugt, wenn n und o gleich 1 ist und m und p gleich 0 ist. Weiterhin ist es für obenstehende Formeln bevorzugt, dass q gleich 0 ist und r gleich 1 ist. Alternativ ist es für obenstehende Formeln bevorzugt, dass q gleich 1 ist und r gleich 0 ist.

Weiterhin ist es für die obenstehenden Formeln bevorzugt, dass mindestens eine der abgebildeten Gruppe R¹, bevorzugt mindestens eine der Gruppen R¹ an der Einheit links vom Stickstoffatom, gewählt ist aus aromatischen oder heteroaromatischen Ringsystemen mit 5 bis 30 aromatischen Ringatomen, die mit einem oder mehreren Resten R² substituiert sein können, besonders bevorzugt aus Aryl- oder Heteroarylgruppen mit 6 bis 16 aromatischen Ringatomen, die mit einem oder mehreren Resten R² substituiert sein können.

Besonders bevorzugt entsprechen Verbindungen der Formel (I) und (II) den folgenden Formeln:

| Formel | Grundkörper | Ar¹ (wahlweise substituiert mit einem oder mehreren R¹) | Ar^{a} (wahlweise substituiert mit einem oder mehreren R¹) | m | n | o | p |
|---|---|---|---|---|---|---|---|
| (I-1-1) | Formel (I-1) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-2-1) | Formel (I-2) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-3-1) | Formel (I-3) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-4-1) | Formel (I-4) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-5-1) | Formel (I-5) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-6-1) | Formel (I-6) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-7-1) | Formel (I-7) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-8-I) | Formel (I-8) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-9-1) | Formel (I-9) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ( "-18 ) | " | " | - | 1 | 0 | 0 | 1 |
| ("-19) | " | " | - | 0 | 1 | 1 | 0 |
| ("-20) | " | " | - | 0 | 1 | 0 | 1 |
| (I-10-1) | Formel (I-10) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-11-1) | Formel (1-11) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (I-12-1) | Formel (I-12) | Phenyl | - | 1 | 0 | 1 | 0 |
| ("-2) | " | " | - | 1 | 0 | 0 | 1 |
| ("-3) | " | " | - | 0 | 1 | 1 | 0 |
| ("-4) | " | " | - | 0 | 1 | 0 | 1 |
| ("-5) | " | Naphthyl | - | 1 | 0 | 1 | 0 |
| ("-6) | " | " | - | 1 | 0 | 0 | 1 |
| ("-7) | " | " | - | 0 | 1 | 1 | 0 |
| ("-8) | " | " | - | 0 | 1 | 0 | 1 |
| ("-9) | " | Fluorenyl | - | 1 | 0 | 1 | 0 |
| ("-10) | " | " | - | 1 | 0 | 0 | 1 |
| ("-11) | " | " | - | 0 | 1 | 1 | 0 |
| ("-12) | " | " | - | 0 | 1 | 0 | 1 |
| ("-13) | " | Dibenzofuranyl | - | 1 | 0 | 1 | 0 |
| ("-14) | " | " | - | 1 | 0 | 0 | 1 |
| ("-15) | " | " | - | 0 | 1 | 1 | 0 |
| ("-16) | " | " | - | 0 | 1 | 0 | 1 |
| ("-17) | " | Dibenzothiophenyl | - | 1 | 0 | 1 | 0 |
| ("-18) | " | " | - | 1 | 0 | 0 | 1 |
| ("-19) | " | " | - | 0 | 1 | 1 | 0 |
| ("-20) | " | " | - | 0 | 1 | 0 | 1 |
| (II-1-1) | Formel (II-1) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-2-1) | Formel (II-2) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " . | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-3-1) | Formel (II-3) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-4-1) | Formel (II-4) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-5-1) | Formel (II-5) | Phenyl | Phenyl | 1 | 0 | 1 | 0 |
| ("-2) | " | " | " | 1 | 0 | 0 | 1 |
| ("- 3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21 ) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-6-1) | Formel (II-6) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| "-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-7-1) | Formel (II-7) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| (II-8-I) | Formel (II-8) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-9-1) | Formel (II-9) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11 ) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| (II-10-1) | Formel (II-10) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-11-1) | Formel (II-11) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ("-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ("-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | | | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |
| (II-12-1) | Formel (II-12) | Phenyl | Phenyl | 1 | 0 | 1 | 0 |
| ("-2) | " | " | " | 1 | 0 | 0 | 1 |
| ("-3) | " | " | " | 0 | 1 | 1 | 0 |
| ("-4) | " | " | " | 0 | 1 | 0 | 1 |
| ("-5) | " | " | Naphthyl | 1 | 0 | 1 | 0 |
| ("-6) | " | " | " | 1 | 0 | 0 | 1 |
| ("-7) | " | " | " | 0 | 1 | 1 | 0 |
| ( "-8) | " | " | " | 0 | 1 | 0 | 1 |
| ("-9) | " | Naphthyl | Phenyl | 1 | 0 | 1 | 0 |
| ("-10) | " | " | " | 1 | 0 | 0 | 1 |
| ( "-11) | " | " | " | 0 | 1 | 1 | 0 |
| ("-12) | " | " | " | 0 | 1 | 0 | 1 |
| ("-13) | " | " | Naphthyl | 1 | 0 | 1 | 0 |
| ("-14) | " | " | " | 1 | 0 | 0 | 1 |
| ("-15) | " | " | " | 0 | 1 | 1 | 0 |
| ("-16) | " | " | " | 0 | 1 | 0 | 1 |
| ("-17) | " | Fluorenyl | Phenyl | 1 | 0 | 1 | 0 |
| ("-18) | " | " | " | 1 | 0 | 0 | 1 |
| ("-19) | " | " | " | 0 | 1 | 1 | 0 |
| ("-20) | " | " | " | 0 | 1 | 0 | 1 |
| ("-21) | " | " | Naphthyl | 1 | 0 | 1 | 0 |
| ("-22) | " | " | " | 1 | 0 | 0 | 1 |
| ("-23) | " | " | " | 0 | 1 | 1 | 0 |
| ("-24) | " | " | " | 0 | 1 | 0 | 1 |
| ("-25) | " | Dibenzofuranyl | Phenyl | 1 | 0 | 1 | 0 |
| ("-26) | " | " | " | 1 | 0 | 0 | 1 |
| ("-27) | " | " | " | 0 | 1 | 1 | 0 |
| ("-28) | " | " | " | 0 | 1 | 0 | 1 |
| ("-29) | " | " | Naphthyl | 1 | 0 | 1 | 0 |
| ("-30) | " | " | " | 1 | 0 | 0 | 1 |
| ("-31) | " | " | " | 0 | 1 | 1 | 0 |
| ("-32) | " | " | " | 0 | 1 | 0 | 1 |
| ("-33) | " | Dibenzothiophenyl | Phenyl | 1 | 0 | 1 | 0 |
| ("-34) | " | " | " | 1 | 0 | 0 | 1 |
| ("-35) | " | " | " | 0 | 1 | 1 | 0 |
| ("-36) | " | " | " | 0 | 1 | 0 | 1 |
| ("-37) | " | " | Naphthyl | 1 | 0 | 1 | 0 |
| ("-38) | " | " | " | 1 | 0 | 0 | 1 |
| ("-39) | " | " | " | 0 | 1 | 1 | 0 |
| ("-40) | " | " | " | 0 | 1 | 0 | 1 |

Besonders bevorzugt entsprechen Verbindungen der Formel (III) den folgenden Formeln:

| Formel | Grundkörper | Ar¹ (wahlweise substituiert mit einem oder mehreren R¹) | m | n | o | p | q | r |
|---|---|---|---|---|---|---|---|---|
| (III-1-1) | Formel (III-1) | Phenyl | 1 | 0 | 1 | 0 | 1 | 0 |
| ( "-2) | " | " | 1 | 0 | 0 | 1 | 1 | 0 |
| ("-3) | " | " | 0 | 1 | 1 | 0 | 1 | 0 |
| ("-4) | " | " | 0 | 1 | 0 | 1 | 1 | 0 |
| ("-5) | " | " | 1 | 0 | 1 | 0 | 0 | 1 |
| ("-6) | " | " | 1 | 0 | 0 | 1 | 0 | 1 |
| ("-7) | " | " | 0 | 1 | 1 | 0 | 0 | 1 |
| ("-8) | " | " | 0 | 1 | 0 | 1 | 0 | 1 |
| ("-9) | " | Naphthyl | 1 | 0 | 1 | 0 | 1 | 0 |
| ("-10) | " | " | 1 | 0 | 0 | 1 | 1 | 0 |
| ("-11) | " | " | 0 | 1 | 1 | 0 | 1 | 0 |
| ("-12) | " | " | 0 | 1 | 0 | 1 | 1 | 0 |
| ("-13) | " | " | 1 | 0 | 1 | 0 | 0 | 1 |
| ("-14) | " | " | 1 | 0 | 0 | 1 | 0 | 1 |
| ( "-15) | " | " | 0 | 1 | 1 | 0 | 0 | 1 |
| ("-16) | " | " | 0 | 1 | 0 | 1 | 0 | 1 |
| ("-17) | " | Fluorenyl | 1 | 0 | 1 | 0 | 1 | 0 |
| ("-18) | " | " | 1 | 0 | 0 | 1 | 1 | 0 |
| ("-19) | " | " | 0 | 1 | 1 | 0 | 1 | 0 |
| ("-20) | " | " | 0 | 1 | 0 | 1 | 1 | 0 |
| ("-21) | " | " | 1 | 0 | 1 | 0 | 0 | 1 |
| ("-22) | " | " | 1 | 0 | 0 | 1 | 0 | 1 |
| ("-23) | " | " | 0 | 1 | 1 | 0 | 0 | 1 |
| ("-24) | " | " | 0 | 1 | 0 | 1 | 0 | 1 |
| ("-25) | " | Dibenzofuranyl | 1 | 0 | 1 | 0 | 1 | 0 |
| ("-26) | " | " | 1 | 0 | 0 | 1 | 1 | 0 |
| ("-27) | " | " | 0 | 1 | 1 | 0 | 1 | 0 |
| ("-28) | " | " | 0 | 1 | 0 | 1 | 1 | 0 |
| ("-29) | " | " | 1 | 0 | 1 | 0 | 0 | 1 |
| ("-30) | " | " | 1 | 0 | 0 | 1 | 0 | 1 |
| ("-31) | " | " | 0 | 1 | 1 | 0 | 0 | 1 |
| ("-32) | " | " | 0 | 1 | 0 | 1 | 0 | 1 |
| ("-33) | " | Dibenzothiophenyl | 1 | 0 | 1 | 0 | 1 | 0 |
| ("-34) | " | " | 1 | 0 | 0 | 1 | 1 | 0 |
| ("-35) | " | " | 0 | 1 | 1 | 0 | 1 | 0 |
| ("-36) | " | " | 0 | 1 | 0 | 1 | 1 | 0 |
| ("-37) | " | " | 1 | 0 | 1 | 0 | 0 | 1 |
| ("-38) | " | " | 1 | 0 | 0 | 1 | 0 | 1 |
| ("-39) | " | " | 0 | 1 | 1 | 0 | 0 | 1 |
| ("-40) | " | " | 0 | 1 | 0 | 1 | 0 | 1 |
| (III-2-1) | Formel (III-2) | Phenyl | 1 | 0 | 1 | 0 | 1 | 0 |
| ("-2) | " | " | 1 | 0 | 0 | 1 | 1 | 0 |
| ("-3) | " | " | 0 | 1 | 1 | 0 | 1 | 0 |
| ("-4) | " | " | 0 | 1 | 0 | 1 | 1 | 0 |
| ("-5) | " | " | 1 | 0 | 1 | 0 | 0 | 1 |
| ("-6) | " | " | 1 | 0 | 0 | 1 | 0 | 1 |
| ("-7) | " | " | 0 | 1 | 1 | 0 | 0 | 1 |
| ("-8) | " | " | 0 | 1 | 0 | 1 | 0 | 1 |
| ("-9) | " | Naphthyl | 1 | 0 | 1 | 0 | 1 | 0 |
| ("-10) | " | " | 1 | 0 | 0 | 1 | 1 | 0 |
| ("-11) | " | " | 0 | 1 | 1 | 0 | 1 | 0 |
| ("-12) | " | " | 0 | 1 | 0 | 1 | 1 | 0 |
| ("-13) | " | " | 1 | 0 | 1 | 0 | 0 | 1 |
| ("-14) | " | " | 1 | 0 | 0 | 1 | 0 | 1 |
| ("-15) | " | " | 0 | 1 | 1 | 0 | 0 | 1 |
| ("-16) | " | " | 0 | 1 | 0 | 1 | 0 | 1 |
| ("-17) | " | Fluorenyl | 1 | 0 | 1 | 0 | 1 | 0 |
| ("-18) | " | " | 1 | 0 | 0 | 1 | 1 | 0 |
| ("-19) | " | " | 0 | 1 | 1 | 0 | 1 | 0 |
| ("-20) | " | " | 0 | 1 | 0 | 1 | 1 | 0 |
| ("-21) | " | " | 1 | 0 | 1 | 0 | 0 | 1 |
| ("-22) | " | " | 1 | 0 | 0 | 1 | 0 | 1 |
| ("-23) | " | " | 0 | 1 | 1 | 0 | 0 | 1 |
| ("-24) | " | " | 0 | 1 | 0 | 1 | 0 | 1 |
| ("-25) | " | Dibenzofuranvl | 1 | 0 | 1 | 0 | 1 | 0 |
| ("-26) | " | " | 1 | 0 | 0 | 1 | 1 | 0 |
| ("-27) | " | " | 0 | 1 | 1 | 0 | 1 | 0 |
| ("-28) | " | " | 0 | 1 | 0 | 1 | 1 | 0 |
| ("-29) | " | " | 1 | 0 | 1 | 0 | 0 | 1 |
| ("-30) | " | " | 1 | 0 | 0 | 1 | 0 | 1 |
| ("-31) | " | " | 0 | 1 | 1 | 0 | 0 | 1 |
| ("-32) | " | " | 0 | 1 | 0 | 1 | 0 | 1 |
| ("-33) | " | Dibenzothiophenyl | 1 | 0 | 1 | 0 | 1 | 0 |
| ("-34) | " | " | 1 | 0 | 0 | 1 | 1 | 0 |
| ("-35) | " | " | 0 | 1 | 1 | 0 | 1 | 0 |
| ("-36) | " | " | 0 | 1 | 0 | 1 | 1 | 0 |
| ("-37) | " | " | 1 | 0 | 1 | 0 | 0 | 1 |
| ("-38) | " | " | 1 | 0 | 0 | 1 | 0 | 1 |
| ("-39) | " | " | 0 | 1 | 1 | 0 | 0 | 1 |
| ("-40) | " | " | 0 | 1 | 0 | 1 | 0 | 1 |
| (III-3-1) | Formel (III-3) | Phenyl | 1 | 0 | 1 | 0 | 1 | 0 |
| ("-2) | " | " | 1 | 0 | 0 | 1 | 1 | 0 |
| ("-3) | " | " | 0 | 1 | 1 | 0 | 1 | 0 |
| ("-4) | " | " | 0 | 1 | 0 | 1 | 1 | 0 |
| ("-5) | " | " | 1 | 0 | 1 | 0 | 0 | 1 |
| ("-6) | " | " | 1 | 0 | 0 | 1 | 0 | 1 |
| ("-7) | " | " | 0 | 1 | 1 | 0 | 0 | 1 |
| ("-8) | " | " | 0 | 1 | 0 | 1 | 0 | 1 |
| ("-9) | " | Naphthyl | 1 | 0 | 1 | 0 | 1 | 0 |
| ("-10) | " | " | 1 | 0 | 0 | 1 | 1 | 0 |
| ("-11) | " | " | 0 | 1 | 1 | 0 | 1 | 0 |
| ("-12) | " | " | 0 | 1 | 0 | 1 | 1 | 0 |
| ("-13) | " | " | 1 | 0 | 1 | 0 | 0 | 1 |
| ("-14) | " | " | 1 | 0 | 0 | 1 | 0 | 1 |
| ("-15) | " | " | 0 | 1 | 1 | 0 | 0 | 1 |
| ("-16) | " | " | 0 | 1 | 0 | 1 | 0 | 1 |
| ("-17) | " | Fluorenyl | 1 | 0 | 1 | 0 | 1 | 0 |
| ("-18) | " | " | 1 | 0 | 0 | 1 | 1 | 0 |
| ("-19) | " | " | 0 | 1 | 1 | 0 | 1 | 0 |
| ("-20) | " | " | 0 | 1 | 0 | 1 | 1 | 0 |
| ("-21) | " | " | 1 | 0 | 1 | 0 | 0 | 1 |
| ("-22) | " | " | 1 | 0 | 0 | 1 | 0 | 1 |
| ("-23) | " | " | 0 | 1 | 1 | 0 | 0 | 1 |
| ("-24) | " | " | 0 | 1 | 0 | 1 | 0 | 1 |
| ("-25) | " | Dibenzofuranyl | 1 | 0 | 1 | 0 | 1 | 0 |
| ("-26) | " | " | 1 | 0 | 0 | 1 | 1 | 0 |
| ("-27) | " | " | 0 | 1 | 1 | 0 | 1 | 0 |
| ("-28) | " | " | 0 | 1 | 0 | 1 | 1 | 0 |
| ("-29) | " | " | 1 | 0 | 1 | 0 | 0 | 1 |
| ("-30) | " | " | 1 | 0 | 0 | 1 | 0 | 1 |
| ("-31) | " | " | 0 | 1 | 1 | 0 | 0 | 1 |
| ("-32) | " | " | 0 | 1 | 0 | 1 | 0 | 1 |
| ("-33) | " | Dibenzothiophenyl | 1 | 0 | 1 | 0 | 1 | 0 |
| ("-34) | " | " | 1 | 0 | 0 | 1 | 1 | 0 |
| ("-35) | " | " | 0 | 1 | 1 | 0 | 1 | 0 |
| ("-36) | " | " | 0 | 1 | 0 | 1 | 1 | 0 |
| ("-37) | " | " | 1 | 0 | 1 | 0 | 0 | 1 |
| ("-38) | " | " | 1 | 0 | 0 | 1 | 0 | 1 |
| ("-39) | " | " | 0 | 1 | 1 | 0 | 0 | 1 |
| ("-40) | " | " | 0 | 1 | 0 | 1 | 0 | 1 |
| (III-4-1) | Formel (III-4) | Phenyl | 1 | 0 | 1 | 0 | 1 | 0 |
| ("-2) | " | " | 1 | 0 | 0 | 1 | 1 | 0 |
| ("-3) | " | " | 0 | 1 | 1 | 0 | 1 | 0 |
| ("-4) | " | " | 0 | 1 | 0 | 1 | 1 | 0 |
| ("-5) | " | " | 1 | 0 | 1 | 0 | 0 | 1 |
| ("-6) | " | " | 1 | 0 | 0 | 1 | 0 | 1 |
| ("-7) | " | " | 0 | 1 | 1 | 0 | 0 | 1 |
| ("-8) | " | " | 0 | 1 | 0 | 1 | 0 | 1 |
| ("-9) | " | Naphthyl | 1 | 0 | 1 | 0 | 1 | 0 |
| ("-10) | " | " | 1 | 0 | 0 | 1 | 1 | 0 |
| ("-11) | " | " | 0 | 1 | 1 | 0 | 1 | 0 |
| ("-12) | " | " | 0 | 1 | 0 | 1 | 1 | 0 |
| ("-13) | " | 94 | 1 | 0 | 1 | 0 | 0 | 1 |
| ("-14) | " | " | 1 | 0 | 0 | 1 | 0 | 1 |
| ("-15) | " | " | 0 | 1 | 1 | 0 | 0 | 1 |
| ("-16) | " | " | 0 | 1 | 0 | 1 | 0 | 1 |
| ("-17) | " | Fluorenyl | 1 | 0 | 1 | 0 | 1 | 0 |
| ("-18) | " | " | 1 | 0 | 0 | 1 | 1 | 0 |
| ("-19) | " | " | 0 | 1 | 1 | 0 | 1 | 0 |
| ("-20) | " | " | 0 | 1 | 0 | 1 | 1 | 0 |
| ("-21) | " | " | 1 | 0 | 1 | 0 | 0 | 1 |
| ("-22) | " | " | 1 | 0 | 0 | 1 | 0 | 1 |
| ("-23) | " | " | 0 | 1 | 1 | 0 | 0 | 1 |
| ("-24) | " | " | 0 | 1 | 0 | 1 | 0 | 1 |
| ("-25) | " | Dibenzofuranyl | 1 | 0 | 1 | 0 | 1 | 0 |
| ("-26) | " | " | 1 | 0 | 0 | 1 | 1 | 0 |
| ("-27) | " | " | 0 | 1 | 1 | 0 | 1 | 0 |
| ("-28) | " | " | 0 | 1 | 0 | 1 | 1 | 0 |
| ("-29) | " | " | 1 | 0 | 1 | 0 | 0 | 1 |
| ("-30) | " | " | 1 | 0 | 0 | 1 | 0 | 1 |
| ("-31) | " | " | 0 | 1 | 1 | 0 | 0 | 1 |
| ("-32) | " | " | 0 | 1 | 0 | 1 | 0 | 1 |
| ("-33) | " | Dibenzothiophenyl | 1 | 0 | 1 | 0 | 1 | 0 |
| ("-34) | " | " | 1 | 0 | 0 | 1 | 1 | 0 |
| ("-35) | " | " | 0 | 1 | 1 | 0 | 1 | 0 |
| ("-36) | " | " | 0 | 1 | 0 | 1 | 1 | 0 |
| ("-37) | " | " | 1 | 0 | 1 | 0 | 0 | 1 |
| ("-38) | " | " | 1 | 0 | 0 | 1 | 0 | 1 |
| ("-39) | " | " | 0 | 1 | 1 | 0 | 0 | 1 |
| ("-40) | " | " | 0 | 1 | 0 | 1 | 0 | 1 |

Beispiele für erfindungsgemäße Verbindungen sind in der folgenden Tabelle aufgeführt.

| | | |
|---|---|---|
| 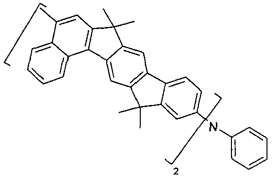 | 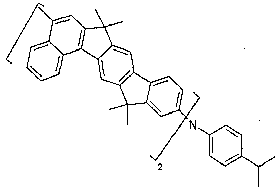 | 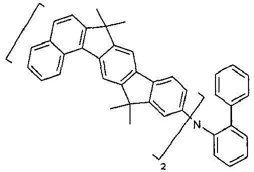 |
| (1) | (2) | (3) |
| 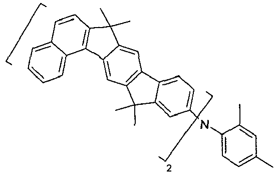 | 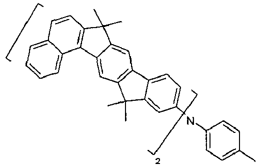 | 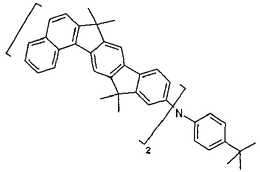 |
| (4) | (5) | (6) |
| 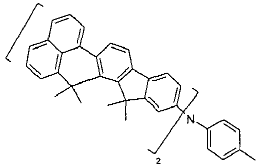 | 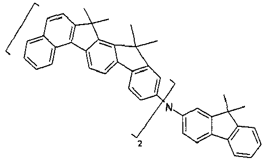 | 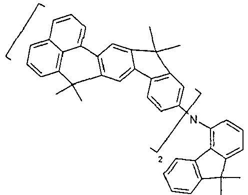 |
| (7) | (8) | (9) |
| 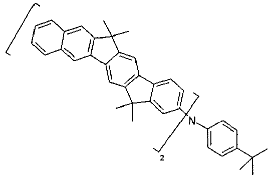 | 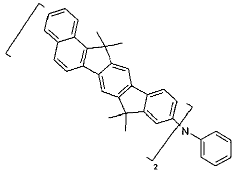 | 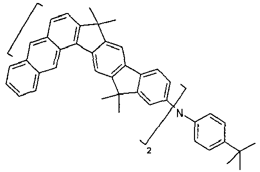 |
| (10) | (11) | (12) |
| 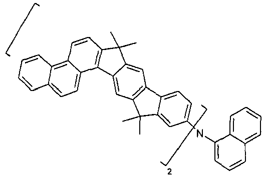 | 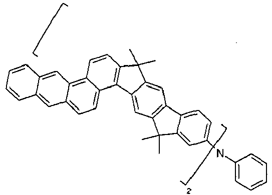 | 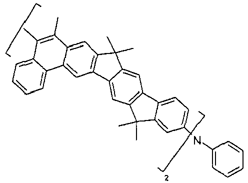 |
| (13) | (14) | (15) |
| 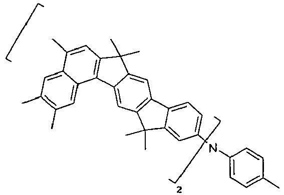 | 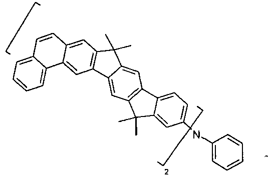 | 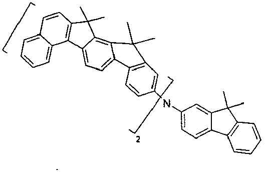 |
| (16) | (17) | (18) |
| 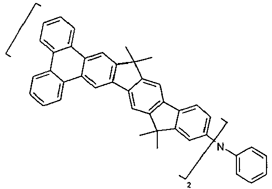 | 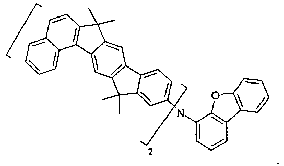 | 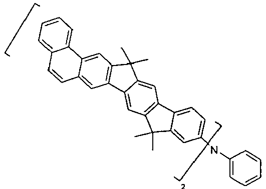 |
| (19) | (20) | (21) |
| 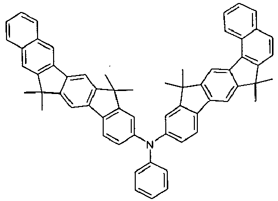 | 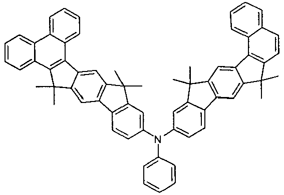 | 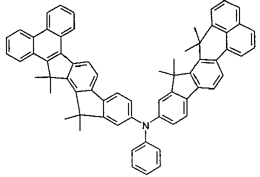 |
| (22) | (23) | (24) |
| 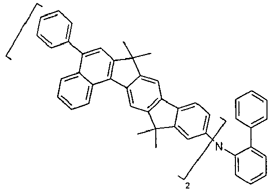 | 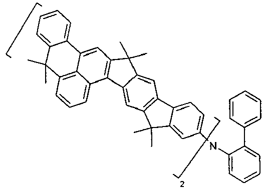 | 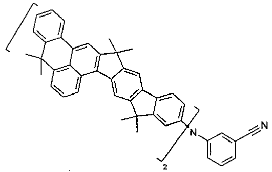 |
| (25) | (26) | (27) |
| 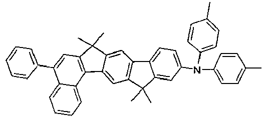 | 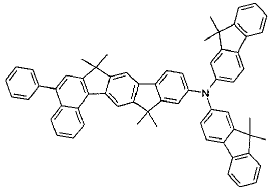 | 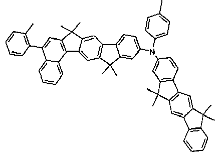 |
| (28) | (29) | (30) |
| 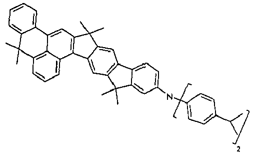 | 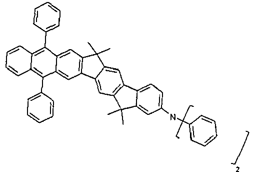 | 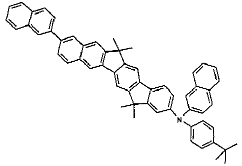 |
| (31) | (32) | (33) |
| 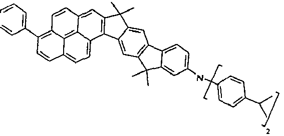 | 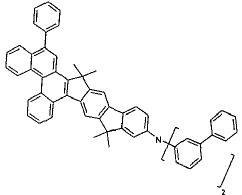 | 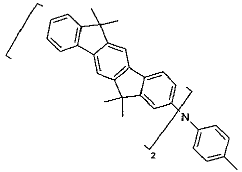 |
| (34) | (35) | (36) |
| 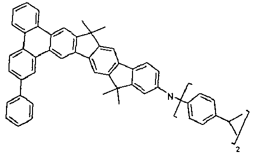 | 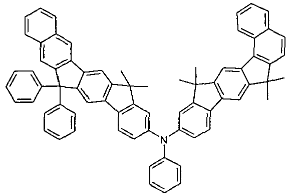 | 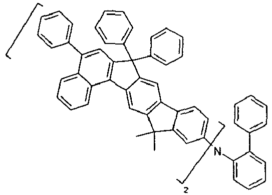 |
| (37) | (38) | (39) |
| 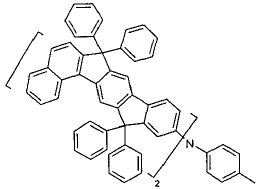 | 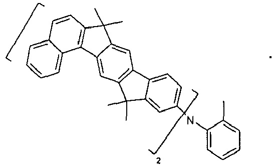 | 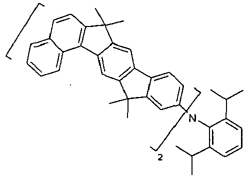 |
| (40) | (41) | (42) |
| 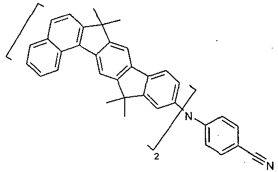 | 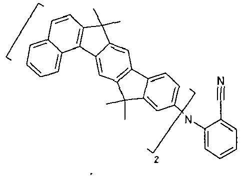 | 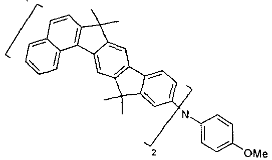 |
| (43) | (44) | (45) |
| 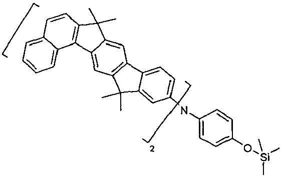 | 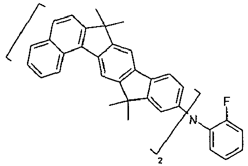 | 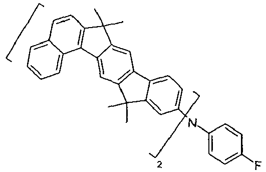 |
| (46) | (47) | (48) |
| 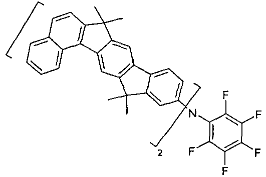 | 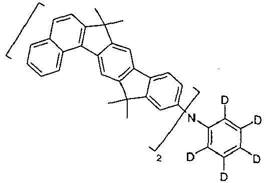 | 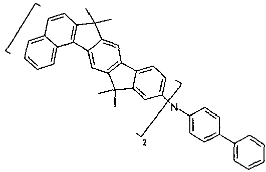 |
| (49) | (50) | (51) |
| 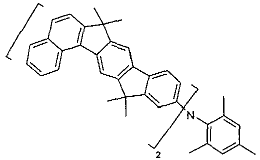 | 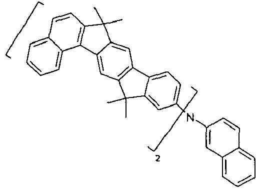 | 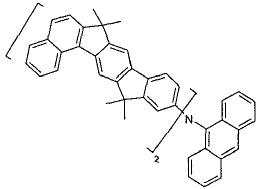 |
| (52) | (53) | (54) |
| 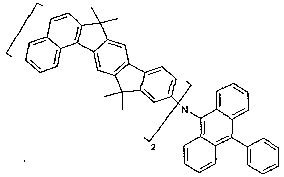 | 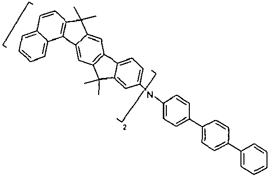 | 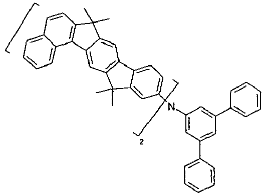 |
| (55) | (56) | (57) |
| 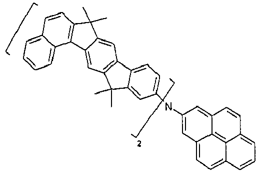 | 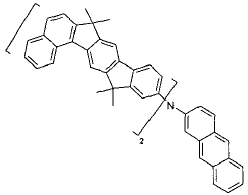 | 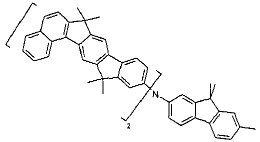 |
| (58) | (59) | (60) |
| 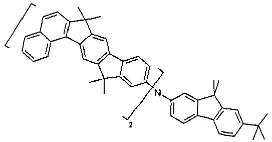 | 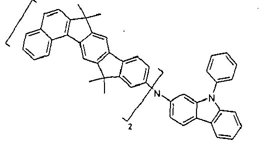 | 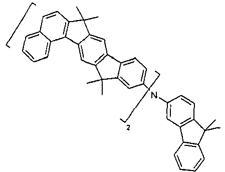 |
| (61) | (62) | (63) |
| 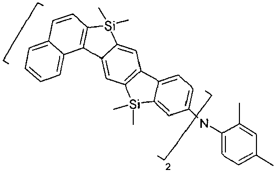 | 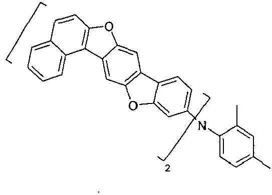 | 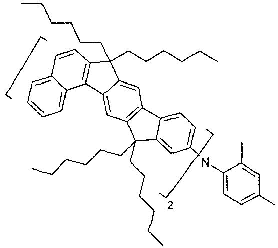 |
| (64) | (65) | (66) |
| 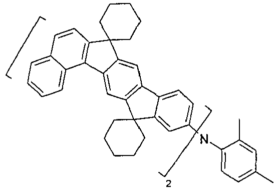 | 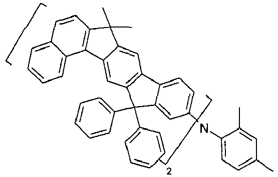 | 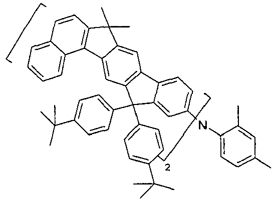 |
| (67) | (68) | (69) |
| 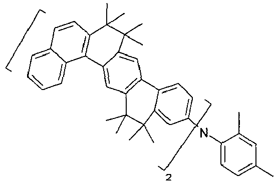 | 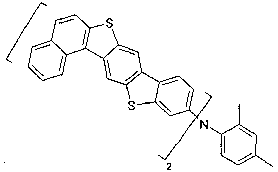 | 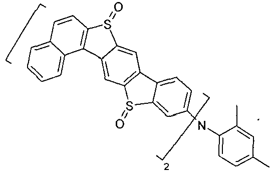 |
| (70) | (71) | (72) |
| 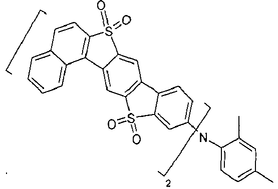 | 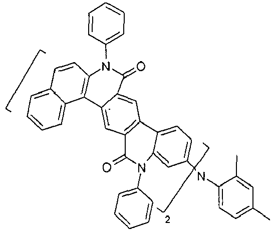 | 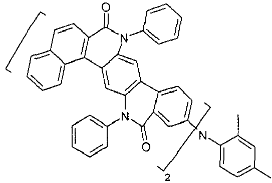 |
| (73) | (74) | (75) |
| 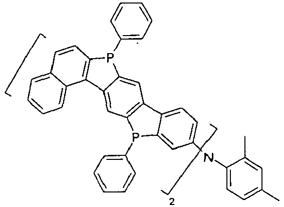 | 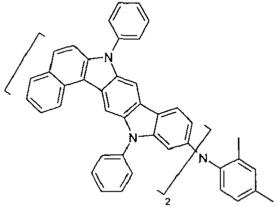 | 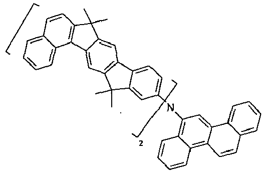 |
| (76) | (77) | (78) |
| 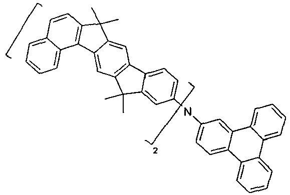 | 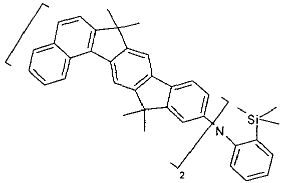 | 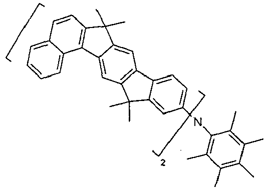 |
| (79) | (80) | (81) |

Die Synthese der erfindungsgemäßen Verbindungen kann gemäß dem Fachmann allgemein bekannten Verfahren der organischen präparativen Chemie erfolgen. Beispiele für bevorzugt eingesetzte Reaktionen sind Halogenierungen sowie übergangsmetallkatalysierte Kupplungsreaktionen, bevorzugt Suzuki-Kupplungen und Buchwald-Kupplungen.

Im Folgenden werden beispielhafte allgemeine Synthesewege zur Herstellung der Verbindungen vorgestellt.

Im Verfahren nach Schema 1 wird von einer Triarylamino-Verbindung ausgegangen, welche reaktive Gruppen an zwei der drei Arylgruppen aufweist. In diesen Positionen kann über eine Übergangsmetallkatalysierte Reaktion, beispielsweise eine Suzuki-Reaktion, eine Bi-Arylverbindung gekuppelt werden, welche Vorstufen zur Bildung von Brücken X trägt. In einer anschließenden Zyklisierungsreaktion werden die Brücken X eingeführt. Es können weitere Funktionalisierungsreaktionen durchgeführt werden, um zur endgültigen erfindungsgemäßen Verbindung zu gelangen.

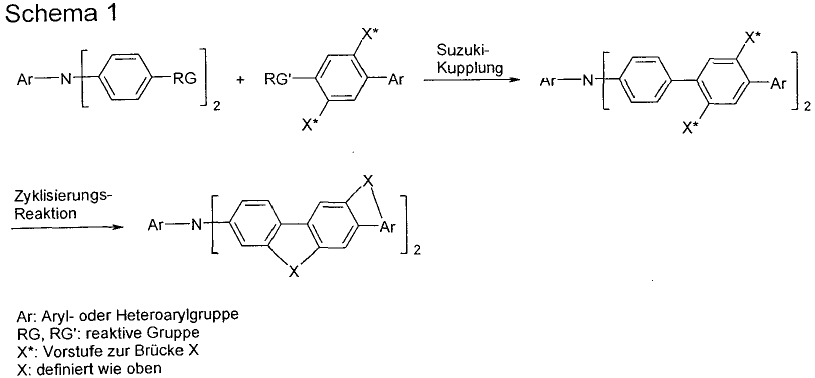

Im Verfahren nach Schema 2 wird von einer verbrückten Tris-Arylverbindung, bevorzugt einer Indenofluorenverbindung ausgegangen. Diese wird in einem ersten Schritt funktionalisiert, beispielsweise durch Bromierung. Anschließend wird durch übergangsmetallkatalysierte Kupplungsreaktion eine Gruppe Ar eingeführt, beispielsweise durch Suzuki-Kupplung. Nach einer erneuten selektiven Funktionalisierung an der gegenüberliegenden Seite des Grundkörpers kann über eine weitere Kupplungsreaktion, beispielsweise eine Buchwald-Kupplung, eine Diarylaminogruppe eingeführt werden. Es können weitere Funktionalisierungsreaktionen durchgeführt werden, um zur endgültigen erfindungsgemäßen Verbindung zu gelangen.

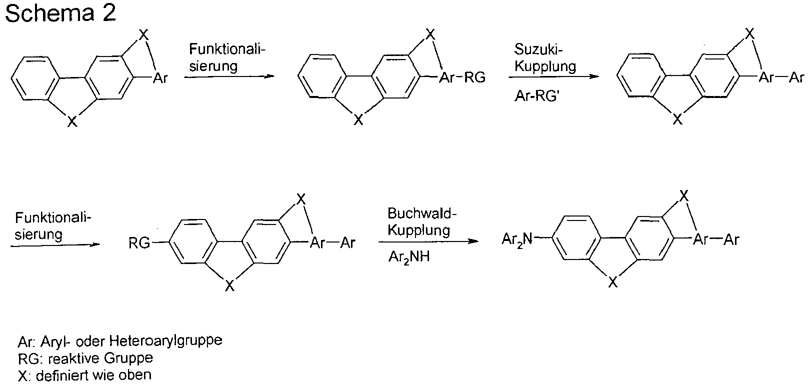

In einem Verfahren nach Schema 3 wird zunächst das Indenofluoren-Derivat hergestellt, dann funktionalisiert, und anschließend in zwei Buchwald-Kupplungen umgesetzt, wobei jeweils verschiedene Amin-Derivate eingesetzt werden. Auf diese Weise können Verbindungen mit drei unterschiedlichen Gruppen am zentralen Stickstoffatom erhalten werden, insbesondere Verbindungen der Formel (III).

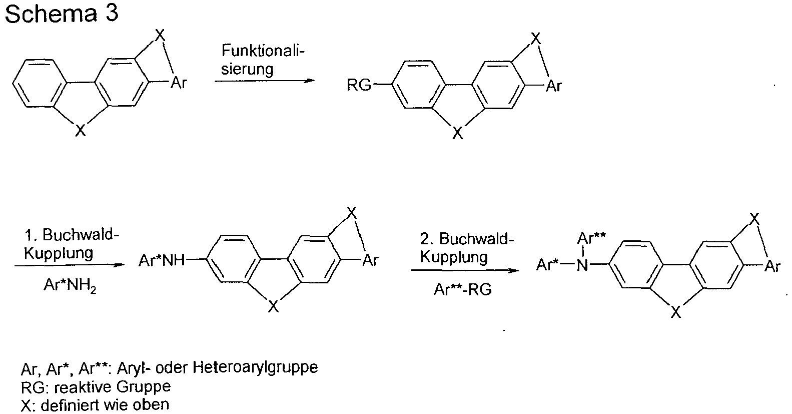

Gegenstand der Erfindung ist damit weiterhin ein Verfahren zur Herstellung einer Verbindung gemäß Formel (I), (II) oder (III), dadurch gekennzeichnet, dass eine oder mehrere metallorganische Kupplungsverfahren eingesetzt werden.

Bevorzugt sind Kupplungsverfahren gewählt aus Buchwald-Kupplungen und Suzuki-Kupplungen.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), (II) oder (III), wobei die Bindung(en) zum Polymer, Oligomer oder Dendrimer an beliebigen, in Formel (I), (II) oder (III) mit R¹ oder R² substituierten Positionen lokalisiert sein können. Je nach Verknüpfung der Verbindung gemäß Formel (I), (II) oder (III) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I), (II) oder (III) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I), (II) oder (III) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I), (II) oder (III) in Oligomeren, Dendrimeren und Polymeren gelten dieselben Bevorzugungen wie oben für Verbindungen gemäß Formel (I), (II) oder (III) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I), (II) oder (III) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I), (II) oder (III) oder mindestens ein Polymer, Oligomer oder Dendrimer enthaltend mindestens eine Einheit gemäß Formel (I), (II) oder (III) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen gemäß Formel (I), (II) oder (III) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weitere Gegenstände der Erfindung sind daher die Verwendung der Verbindungen gemäß Formel (I), (II) oder (III) in elektronischen Vorrichtungen sowie elektronische Vorrichtungen selbst, welche eine oder mehrere Verbindungen gemäß Formel (I), (II) oder (III) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Besonders bevorzugt sind organische Elektrolumineszenzvorrichtungen, enthaltend Anode, Kathode und mindestens eine emittierende Schicht, dadurch gekennzeichnet, dass mindestens eine organische Schicht mindestens eine Verbindung gemäß Formel (I), (II) oder (III) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der organischen Elektrolumineszenzvorrichtung ist bevorzugt die Folgende:
Anode-Lochinjektionsschicht-Lochtransportschicht-emittierende Schicht-Elektronentransportschicht-Elektroneninjektionsschicht-Kathode.
Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I), (II) oder (III) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in der Lochtransportschicht oder in einer anderen Schicht vorhanden sein.
Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist bevorzugt, wenn die Verbindung gemäß Formel (I), (II) oder (III) in einer emittierenden Schicht eingesetzt wird. Insbesondere eignet sich die Verbindung gemäß Formel (I), (II) oder (III) zur Verwendung als emittierendes Material (Dotand).

Die erfindungsgemäße Verbindung eignet sich besonders zur Verwendung als blau emittierende Emitterverbindung. Dabei kann die betreffende elektronische Vorrichtung eine einzige emittierende Schicht enthaltend die erfindungsgemäße Verbindung enthalten, oder sie kann zwei oder mehr emittierende Schichten enthalten. Die weiteren emittierenden Schichten können dabei eine oder mehrere erfindungsgemäße Verbindungen oder alternativ andere Verbindungen enthalten.

Wenn die erfindungsgemäße Verbindung als emittierendes Material in einer emittierenden Schicht eingesetzt wird, wird sie bevorzugt in Kombination mit einem oder mehreren Hostmaterialien eingesetzt. Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil der erfindungsgemäßen Verbindung in der Mischung der emittierenden Schicht beträgt zwischen 0.1 und 50.0 Vol.-%, bevorzugt zwischen 0.5 und 20.0 Vol.-%, besonders bevorzugt zwischen 1.0 und 10.0 Vol.-%. Entsprechend beträgt der Anteil des Hostmaterials bzw. der Hostmaterialien zwischen 50.0 und 99.9 Vol.-%, bevorzugt zwischen 80.0 und 99.5 Vol.-%, besonders bevorzugt zwischen 90.0 und 99.0 Vol.-%.

Bevorzugte Hostmaterialien (Matrixmaterialien) zur Verwendung in Kombination mit den erfindungsgemäßen Materialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Je nach Substitutionsmuster können die erfindungsgemäßen Verbindungen auch in anderen Schichten eingesetzt werden, beispielsweise als Lochtransportmaterialien in einer Lochinjektions- oder Lochtransportschicht oder als Hostmaterialien in einer emittierenden Schicht, bevorzugt als Hostmaterialien für phosphoreszierende Emitter.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Dotanden sind neben den erfindungsgemäßen Verbindungen ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Bevorzugte Matrixmaterialien zur Verwendung mit fluoreszierenden Dotanden sind obenstehend aufgeführt.

Bevorzugte Matrixmaterialien für phosphoreszierende Dotanden sind aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind Indenofluorenamine und Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449) oder Dibenzoindenofluorenamine (z. B. gemäß WO 07/140847). Weiterhin geeignete Lochtransport- und Lochinjektionsmaterialien sind Derivate der oben abgebildeten Verbindungen, wie sie in JP 2001/226331, EP 676461, EP 650955, WO 01/049806, US 4780536, WO 98/30071, EP 891121, EP 1661888, JP 2006/253445, EP 650955, WO 06/073054 und US 5061569 offenbart werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z.B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I), (II) oder (III) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

Die folgenden Ausführungsbeispiele dienen zur Verdeutlichung und Erläuterung der Erfindung.

### Ausführungsbeispiele

### A) Synthesebeispiele:

4-Bromtoluol und Diphenylamin sind kommerziell erhältlich. Die Synthese von 2-Chlor-5-naphthalin-1-yl-terephthalsäurediethylester ist in WO 2010/012328 A1 beschrieben.

### A-1) Variante I

### Synthese von 7,7,13,13-Tetramethyl-N-(7,7,13,13-tetramethyl-7,13-di-hydrobenzo[g]indeno[1,2-b]fluoren-11-yl)-N-(p-tolyl)-7,13-dihydro-benzo[g]indeno[1,2-b]fluoren-11-amin (I)

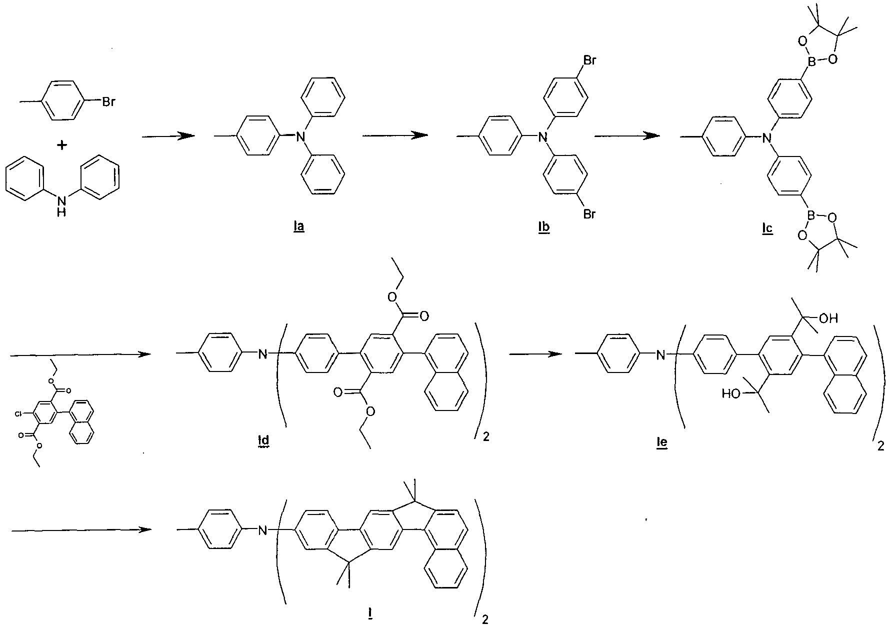

### (4-Methylphenyl)diphenylamin (Ia)

Neben der hier beschriebenen Synthese stehen dem Fachmann weitere, in der Literatur beschriebene Synthesen, zur Verfügung.

Diphenylamin (88.34 g, 520 mmol) und 4-Bromtoluol (82.0g, 470 mmol) werden in 900 mL Toluol gelöst. Anschließend wird die Reaktionslösung mit Tri-*ortho*-tolylphosphin (1.46 g, 4.7 mmol), Palladium(ll)-acetat (0.53 g, 2.4 mmol) und Natrium-*tert*.-butoxid (69.1 g, 700 mmol) versetzt und für 3 Tage unter Rückfluss erhitzt. Die Mischung wird bei Raumtemperatur mit Toluol und dest. H₂O erweitert, die organische Phase abgetrennt und die wässrige Phase mehrmals mit Toluol extrahiert. Die org. Phase wird mit MgSO₄ getrocknet, über AlOx filtriert und eingeengt. Der Rückstand wird mit Heptan zur Präzipitation gebracht und aus Isopropanol umkristallisiert. Man erhält (4-Methylphenyl)diphenylamin als farblosen Feststoff (85.7g, 70% d. Th.).

Analog dazu werden folgende Verbindungen hergestellt:

| Amin | Brom-Aren | Produkt | Ausbeute |
|---|---|---|---|
| 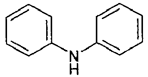 | 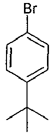 | 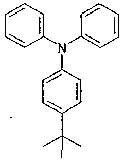 | 65% |
| 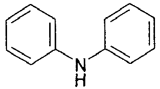 | 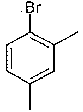 | 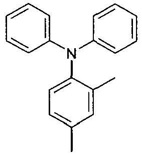 | 75% |
| 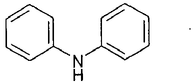 | 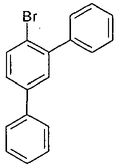 | 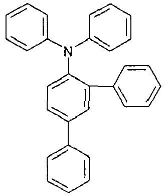 | 62% |
| 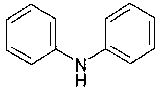 | 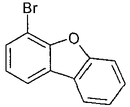 | 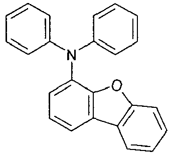 | 68% |

### Bis-(4-brom-phenyl)-p-tolyl-amin (Ib)

(4-Methylphenyl)diphenylamin (85.2 g, 330 mmol) wird in 1 L DCM gelöst und auf 0 °C gekühlt. Unter Rühren wird in kleinen Portionen N-Bromsuccinimid (117 g, 660 mmol) zugegeben, so dass die Reaktionstemperatur nicht über 5 °C steigt. Die Reaktion wird über Nacht im Eisbad auf Raumtemperatur erwärmt. Man gibt nun 500 mL einer 10%igen Na₂SO₃-Lösung hinzu und trennt die Phasen. Die wässrige Phase wird mit DCM mehrmals extrahiert. Die organische Phase wird mit dest. H₂O gewaschen, getrocknet und vom Lösungsmittel befreit. Der erhaltene Feststoff wird mehrmals mit 1-Butanol umkristallisiert. Man erhält 129 g eines farblosen Feststoffes (94% d. Th.).

### 4-Methyl-N,N-bis(4-(4,4,5,5-tetra-methyl-1,3,2-dioxaborolan-2-yl)phenyl-anilin (Ic)

Bis-(4-brom-phenyl)-p-tolyl-amin (128 g, 310 mmol) und Bispinacolatodiboran (195 g, 770 mmol) werden in 1.5 L THF gelöst. Dann werden Kaliumacetat (241 g, 2460 mmol) und 1,1'-Bis(diphenylphosphino)ferrocen-dichloropalladium(ll)*DCM (7.52 g, 9.2 mmol) zu der Reaktionslösung hinzugegeben und für sechs Tage unter Rückfluss erhitzt. Nach dem Abkühlen auf Raumtemperatur wird der Ansatz mit DCM und dest. H₂O erweitert und die wässrige Phase mehrmals mit DCM extrahiert. Die vereinigten organischen Phasen werden mit dest. H₂O gewaschen und nach Trocknen mit MgSO₄ über AlOx filtriert. Das Lösemittel wird bei Normaldruck entfernt. Der erhaltene Feststoff wird mit Heptan und Acetonitril gewaschen. Es werden 110 g eines hellgrauen Pulvers erhalten (70% d. Th.).

### Tetraethyl-4',4"'-(p-tolylazandiyl)bis(4-(naphthalin-1-yl)-[1,1'-biphenyl]-2,5-dicarboxylat) (Id)

4-Methyl-*N,N*-bis(4-(4,4,5,5-tetra-methyl-1,3,2-dioxaborolan-2-yl)phenyl-anilin (110 g, 215 mmol) und 2-Chlor-5-naphthalin-1-yl-terephthalsäurediethylester (189 g, 495 mmol) werden in 1.2L Toluol gelöst und mit Tetrakis-(triphenylphosphin)-palladium (4.97 g, 4.3 mmol) und 2-Dicyclohexylphosphino-2',6'-dimethoxybiphenyl (3.53 g, 8.6 mmol) versetzt. Anschließend gibt man Tetraethylammoniumhydroxid (20%ig in H₂O) (450 mL, 645 mmol) zu der Reaktionslösung. Der Ansatz wird für sieben Stunden unter Rückfluss erhitzt, auf Raumtemperatur abgekühlt und mit dest. H₂O erweitert. Nach Phasentrennung wird die wässrige Phase mehrmals mit Toluol extrahiert. Die vereinigten organischen Phasen werden mit dest. H₂O gewaschen, über MgSO₄ getrocknet und über AlOx filtriert. Die organische Phase wird bis zu einem dunkelorangen zähen Öl eingeengt und chromatographisch aufgereinigt (Kieselgel, Heptan/THF 85:15). 147 g (72% d. Th.) Tetraethyl-4',4"'-(*p*-tolylazan-diyl)bis(4-(naphthalin-1-yl)-[1,1'-biphenyl]-2,5-dicarboxylat) werden in Form eines intensiv gelben Feststoffes isoliert.

Analog dazu werden folgende Verbindungen hergestellt:

| Boronsäureester | Terephthalsäureesterderivat | Produkt | Ausbeute |
|---|---|---|---|
| 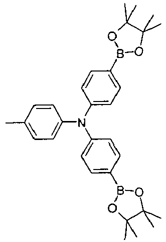 | 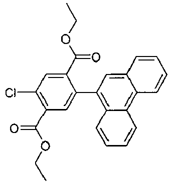 | 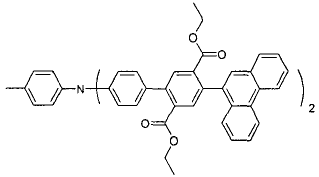 | 69% |
| wie oben | 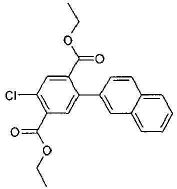 | 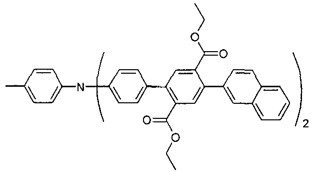 | 76% |
| wie oben | 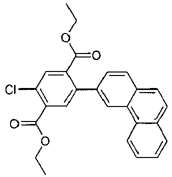 | 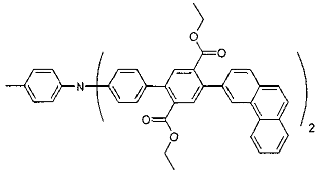 | 74% |
| wie oben | 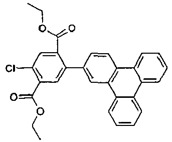 | 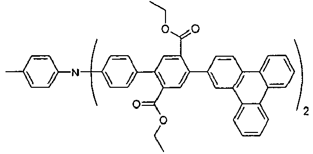 | 68% |

Analog kann die folgende Verbindung hergestellt werden:

| Boronsäureester | Terephthalsäureesterderivat | Produkt |
|---|---|---|
| 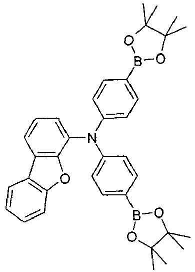 | 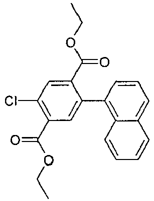 | 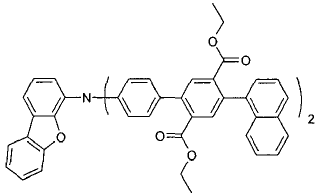 |

### 2-[4'-{[2',5'-Bis-(1-hydroxy-1-methyl-ethyl)-4'-naphthalin-1-yl-biphenyl-4-yl]-p-tolyl-amino}-5-(1-hydroxy-1-methyl-ethyl)-4-naphthalin-1-yl-biphenyl-2-yl]-propan-2-ol (Ie)

Tetraethyl-4',4"'-(*p*-tolylazandiyl)bis(4-(naphthalin-1-yl)-[1,1'-biphenyl]-2,5-dicarboxylat) (147 g, 154 mmol) werden in 750 mL THF gelöst und bei 0 °C mit Methylmagnesiumchlorid (20%ige Lösung in THF) (617 mL, 1700 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. H₂O erweitert und gründlich mit Toluol extrahiert. Die vereinigten organischen Phasen werden mehrmals mit dest. H₂O und einmal mit NaHCO₃-Lösung gewaschen und über MgSO₄ getrocknet. Nach Entfernen des Lösemittels im Vakuum erhält man einen hellbeigen Festoff. Dieser wird mit einem Heptan/Isopropanol-Gemisch gewaschen. Man isoliert 136 g (98% d. Th.) 2-[4'-{[2',5'-Bis-(1-hydroxy-1-methyl-ethyl)-4'-naphthalin-1-yl-biphenyl-4-yl]-p-tolyl-amino}-5-(1-hydroxy-1-methyl-ethyl)-4-naphthalin-1-yl-biphenyl-2-yl]-propan-2-ol als farblosen Feststoff.

### 7,7,13,13-Tetramethyl-N-(7,7,13,13-tetramethyl-7,13-dihydrobenzo[g]indeno[1,2-b]fluoren-11-yl)-N-(p-tolyl)-7,13-dihydrobenzo[g]indeno[1,2-b]fluoren-11-amin (I) (Synthesebeispiel 1)

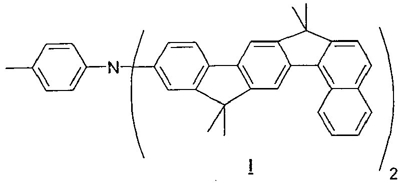

2-[4'-{[2',5'-Bis-(1-hydroxy-1-methyl-ethyl)-4'-naphthalin-1-yl-biphenyl-4-yl]-*p*-tolyl-amino}-5-(1-hydroxy-1-methyl-ethyl)-4-naphthalin-1-yl-biphenyl-2-yl]-propan-2-ol (135 g, 151 mmol) werden mit 1.3 L DCM gelöst und bei -20 °C mit Methansulfonsäure (69 mL, 1060 mmol) und Polyphosphorsäure (156 g, 1350 mmol) versetzt. Man lässt die Reaktionslösung über Nacht auf Raumtemperatur erwärmen. Der ausgefallene gelbe Festoff wird abfiltriert und durch Soxhlett-Extraktion und nachfolgende Sublimation aufgereingt. Man isoliert 47.4 g eines gelben Feststoffes. (38% d. Th.). Analog dazu werden folgende Verbindungen hergestellt:

| SyntheseBeispiel | Struktur | Ausbeute |
|---|---|---|
| 2 | 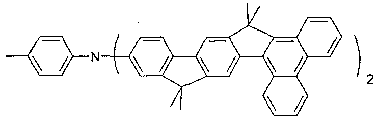 | 44% |
| 3 | 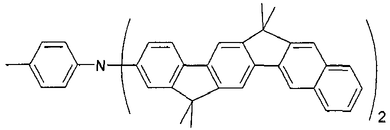 | 42% |
| 4 | 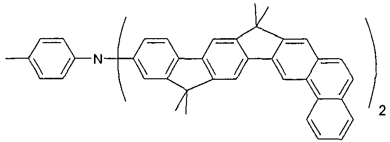 | 39% |
| 5 | 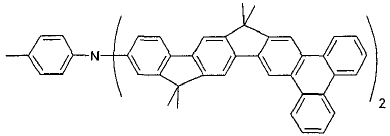 | 34% |

Analog kann die folgende Verbindung hergestellt werden:

| SyntheseBeispiel | Struktur |
|---|---|
| 6 | 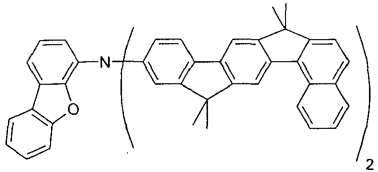 |

### A-2) Variante II

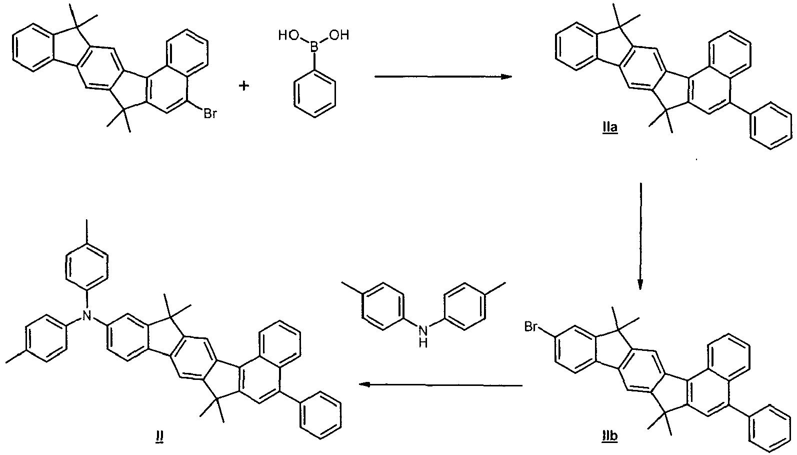

### 7,7,13,13-Tetramethyl-5-phenyl-7,13-dihydrobenzo[g]indeno[1,2-b]fluoren (IIa)

12.8 g (103 mmol) Benzolboronsäure, 37.7 g (86 mmol) 5-Brom-7,7,13,13-tetramethyl-7,13-dihydrobenzo[g]indeno[1,2-b]-flouren und 29.7 g (215 mmol) K₂CO₃ werden in 500 mL Toluol/Wasser (1:1) suspendiert. Zu dieser Suspension werden 0.99 g (0.86 mmol) Tetrakis-(triphenylphosphin)-palladium gegeben, und die Reaktionsmischung wird 16h unter Rückfluss erhitzt. Nach Erkalten wird die Reaktionsmischung mit Essigester verdünnt, die organische Phase abgetrennt, dreimal mit 100 mL Wasser gewaschen und anschließend zur Trockene eingeengt. Nach Filtration des Rohproduktes über Kieselgel mit Toluol wird der verbleibende Rückstand aus Heptan/Toluol umkristallisiert. Die Ausbeute beträgt 29.2 g (78% d. Th).

Analog dazu werden folgende Verbindungen hergestellt:

| Edukt 1 | Edukt 2 | Produkt | Ausbeute |
|---|---|---|---|
| 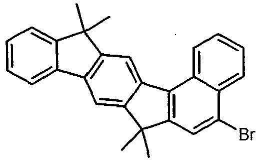 | 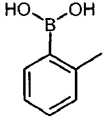 | 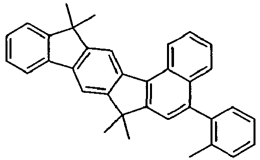 | 74% |
| 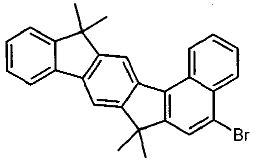 | 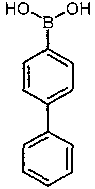 | 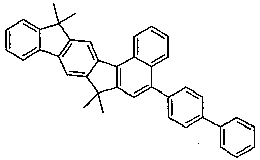 | 69% |
| 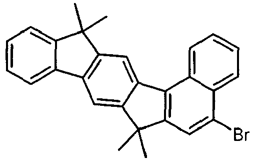 | 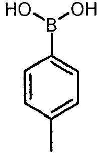 | 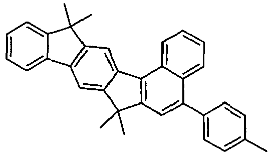 | 79% |
| 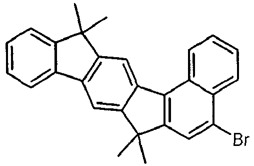 | 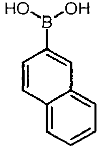 | 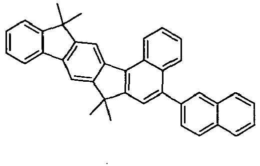 | 73% |
| 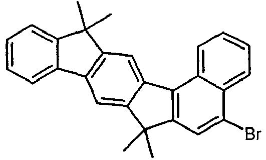 | 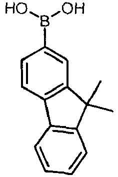 | 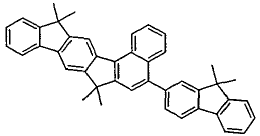 | 67% |
| 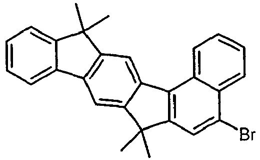 | 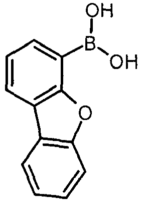 | 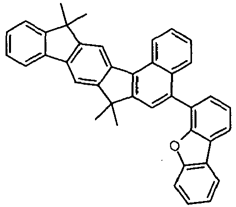 | 59% |
| 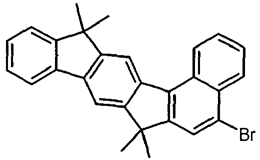 | 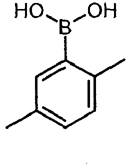 | 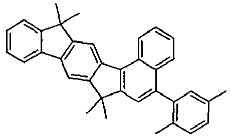 | 81% |

### 11-Brom-7,7,13,13-tetramethyl-5-phenyl-7,13-dihydrobenzo[g]-indeno[1,2-b]fluoren (IIb)

29.2 g (67 mmol) 7,7,13,13-Tetramethyl-5-phenyl-7,13-dihydrobenzo[*g*]indeno[1,2-*b*]fluoren (IIa) werden in 500 mL CHCl₃ gelöst und bei -10°C langsam mit 10.8 g (67 mmol) Brom, gelöst in 500 mL CHCl₃, versetzt. Nach vollständiger Umsetzung wird Wasser dazu gegeben, die organische Phase abgetrennt, getrocknet und eingeengt. Das Rohprodukt wird anschließend mit Heptan/Toluol (5:1) mehrfach heiß ausgerührt. Ausbeute: 30.5 g (89%) des Produktes als weißen Feststoff.

Analog dazu werden folgende Verbindungen hergestellt:

| Edukt 1 | Produkt | Ausbeute |
|---|---|---|
| 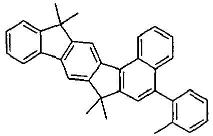 | 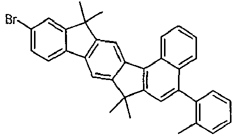 | 83% |

| | | |
|---|---|---|
| 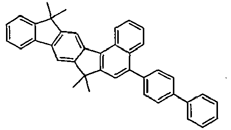 | 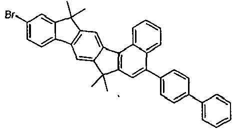 | 75% |
| 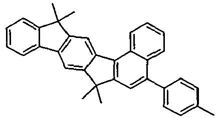 | 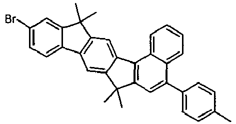 | 81% |
| 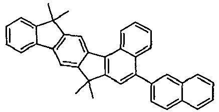 | 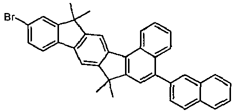 | 62% |
| 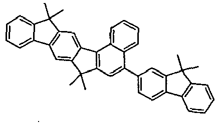 | 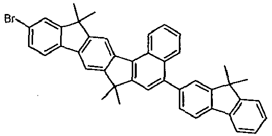 | 72% |
| 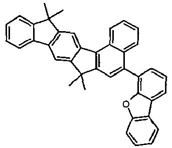 | 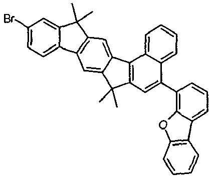 | 44% |
| 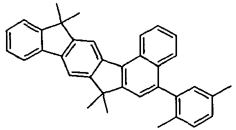 | 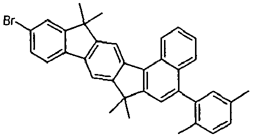 | 87% |

### 7,7,13,13-Tetramethyl-5-phenyl-N,N-di-p-tolyl-7, 13-dihydrobenzo[g]indeno[1,2-b]fluoren-11-amin (II) (Synthesebeispiel 7)

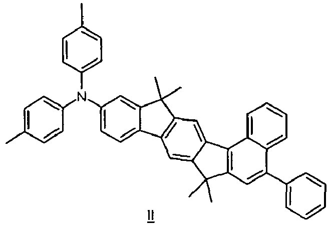

9.74 g Di-p-tolyl-amin (49.4 mmol), 11-Brom-7,7,13,13-tetramethyl-5-phenyl-7,13-dihydrobenzo[*g*]indeno[1,2-*b*]fluoren (IIb) (41.1 mmol) werden in 500 mL Toluol gelöst. Die Lösung wird entgast und mit Argon gesättigt. Danach wird sie mit 2.5 mL (2.5 mmol) einer 1M Tri-*tert*.-Butylphosphin Lösung und 0.355 g (1.23 mmol) Palladium(II)-acetat versetzt. Anschließend werden 11.9 g Natrium-*tert*.-butylat (124 mmol) zugegeben. Die Reaktionsmischung wird für 12h unter Schutzgasatmosphäre zum Sieden erhitzt. Das Gemisch wird im Anschluss mit Wasser versetzt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 15.2 g (58 % d. Th).

Analog dazu werden folgende Verbindungen hergestellt:

| Bsp. | Edukt 1 | Edukt 2 | Produkt | Ausbeute |
|---|---|---|---|---|
| 8 | 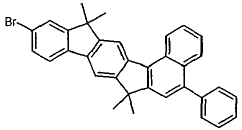 | 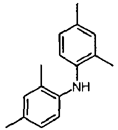 | 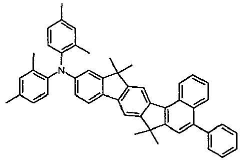 | 69% |
| 9 | 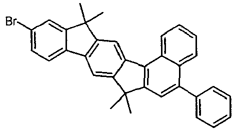 | 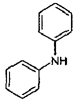 | 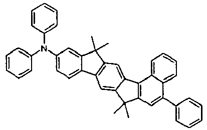 | 78% |
| 10 | 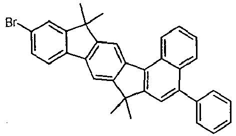 | 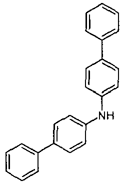 | 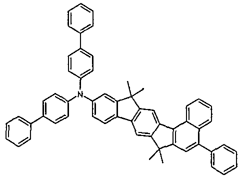 | 72% |
| 11 | 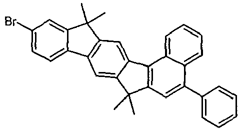 | 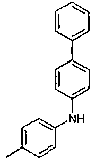 | 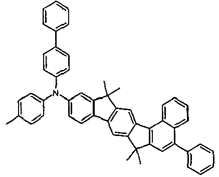 | 83% |
| 12 | 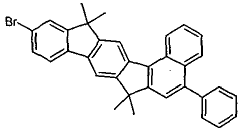 | 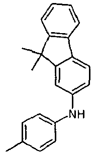 | 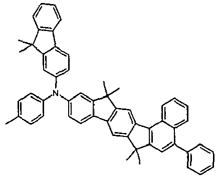 | 76% |
| 13 | 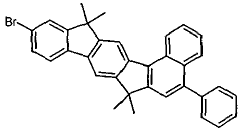 | 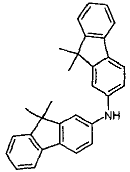 | 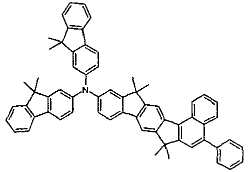 | 67% |
| 14 | 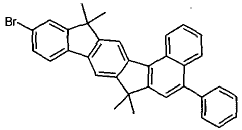 | 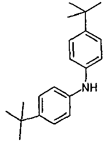 | 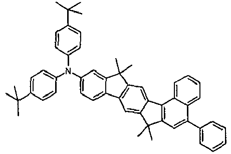 | 75% |
| 15 | 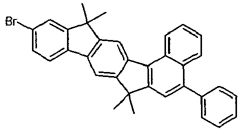 | 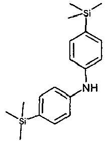 | 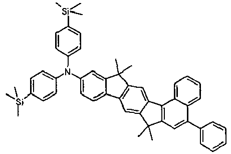 | 81% |
| 16 | 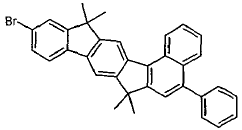 | 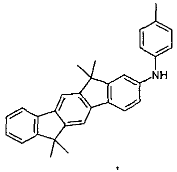 | 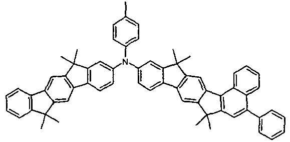 | 58% |
| 17 | 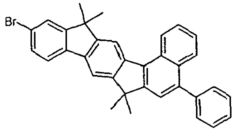 | 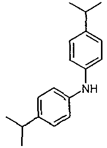 | 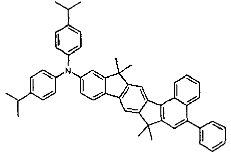 | 73% |
| 18 | 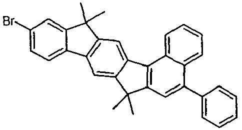 | 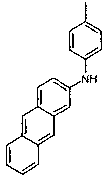 | 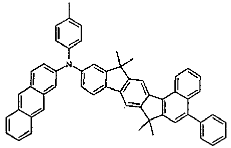 | 61% |
| 19 | 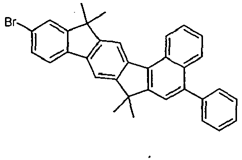 | 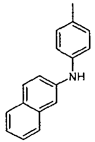 | 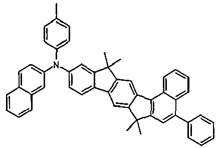 | 68% |
| 20 | 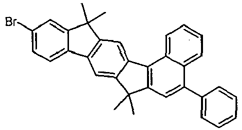 | 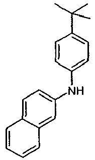 | 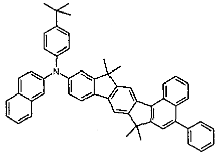 | 72% |
| 21 | 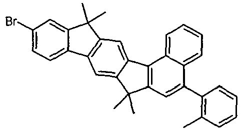 | 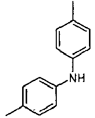 | 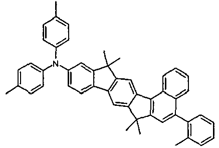 | 81% |
| 22 | 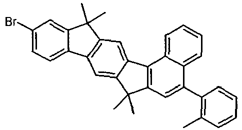 | 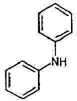 | 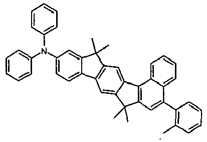 | 79% |
| 23 | 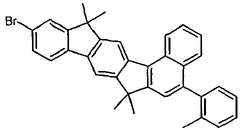 | 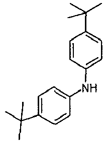 | 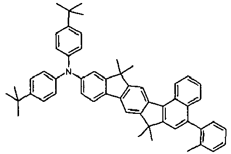 | 77% |
| 24 | 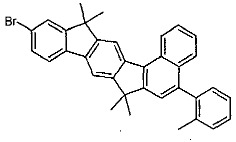 | 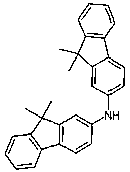 | 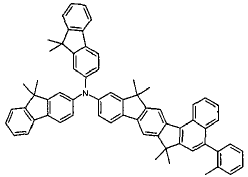 | 64% |
| 25 | 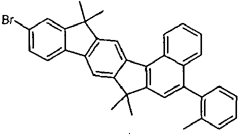 | 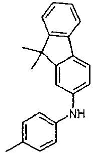 | 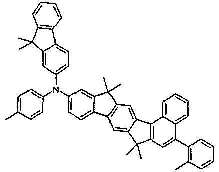 | 77% |
| 26 | 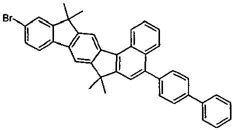 | 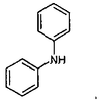 | 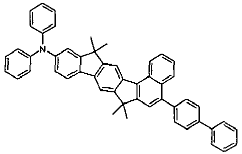 | 84% |
| 27 | 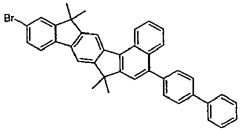 | 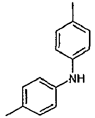 | 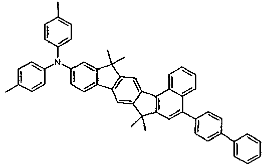 | 72% |
| 28 | 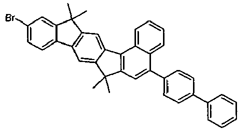 | 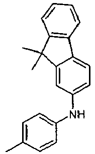 | 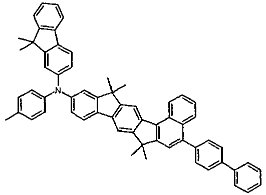 | 68% |
| 29 | 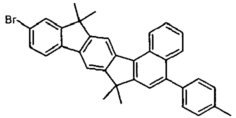 | 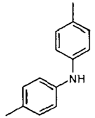 | 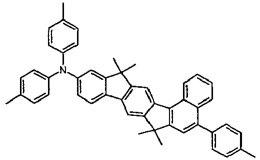 | 81% |
| 30 | 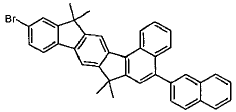 | 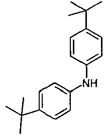 | 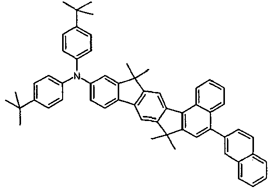 | 79% |
| 31 | 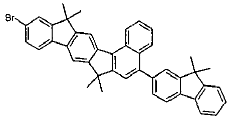 | 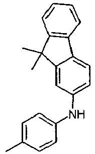 | 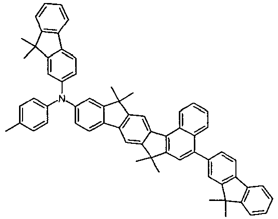 | 73% |
| 32 | 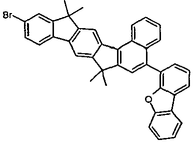 | 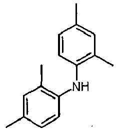 | 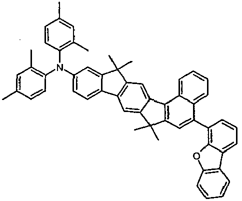 | 77% |
| 33 | 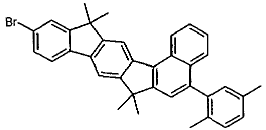 | 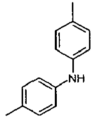 | 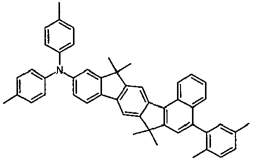 | 82% |
| 34 | 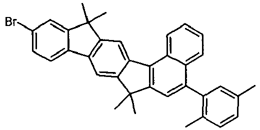 | 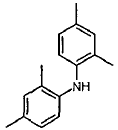 | 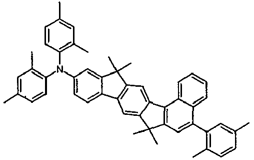 | 77% |
| 35 | 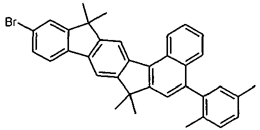 | 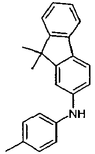 | 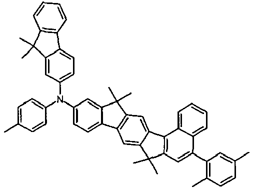 | 65% |
| 36 | 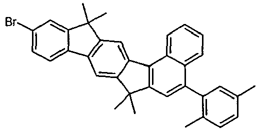 | 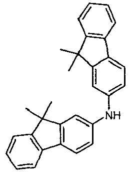 | 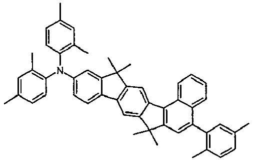 | 79% |

### A-3) Variante III

### III-1) Synthese der Bausteine (i)

### Allgemeines Reaktionsschema:

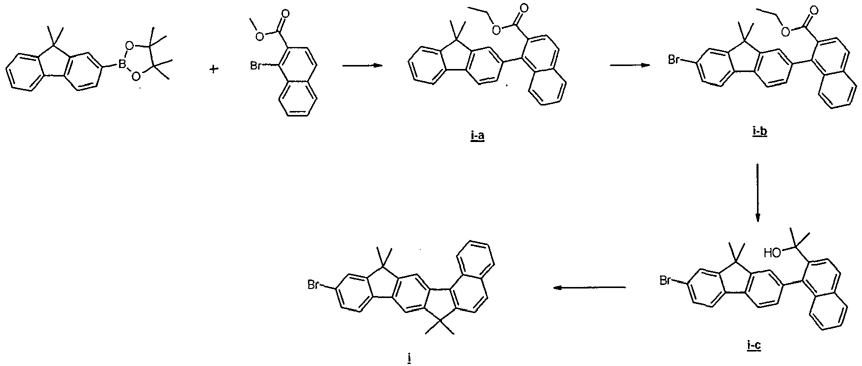

### Ethyl 1-(9,9-dimethyl-9H-fluoren-2-yl)-2-naphthoat (i-a)

1-Brom-naphthalen-2-carboxylsäuremethylester (150 g, 563 mmol), 9,9-dimethyl-9H-fluorene-2-yl-boronester (148.9 g, 619 mmol) und Kaliumphosphatrnonohydrat (286 g, 1.182 mol) werden in einer Mischung von 1.2 L Toluol und 1 L Wasser gelöst und mit Palladiumacetat (1.28 g, 5.6 mmol) und Tri-orthotolyl-Phosphin (3.5 g, 11.3 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über Aluminiumoxid filtriert. Die organische Phase wird bis zu einem orangen Öl eingeengt. Es werden 213 g Produkt erhalten (99% d. Th.).

### Ethyl 1-(7-brom-9,9-dimethyl-9H-fluoren-2-yl)-2-naphthoat (i-b)

**(i-a)** (122 g, 295 mmol) wird in 1 L Chloroform gelöst und auf 0°C gekühlt. Unter Rühren wird eine Dibrom-Lösung (14.4 mL, 280 mmol) in 0.5 L Chloroform zugetropft, so dass die Reaktionstemperatur nicht über 5 °C steigt. Die Reaktion wird über Nacht im Eisbad auf Raumtemperatur erwärmt. 500 mL einer 10%igen Natriumthiosulfat-Lösung werden hinzu gegeben und die Phasen getrennt. Nach Phasentrennung wird die wässrige Phase mehrmals mit Chloroform extrahiert. Die vereinigten organischen Phasen werden mit dest. Wasser gewaschen, über Magnesiumsulfat getrocknet und über Aluminiumoxid filtriert. Die organische Phase wird bis zu einem farblosen Öl eingeengt. Es werden 128 g Produkt erhalten (95 % d. Th.).

### 2-(1-(7-Brom-9,9-dimethyl-9H-fluoren-2-yl)naphthalin-2-yl)propan-2-ol (i-c)

**(i-b)** (80 g, 175 mmol) und Cerium(III) Chlorid (48 g, 247 mmol) werden in 800 mL THF gelöst und bei 0°C mit Methylmagnesiumchlorid (3 M Lösung in THF) (146 mL, 437 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels im Vakuum erhält man einen hellbeigen Feststoff. Dieser wird in einem Heptan/ Toluol-Gemisch umkristallisiert. Man isoliert 69 g (86% d. Th.) als farblosen Feststoff.

### 11-Brom-7,7,13,13-tetramethyl-7,13-dihydrobenzo[g]indeno[1,2-b]fluoren (i)

**(i-c)** (61 g, 133 mmol) wird mit 300 mL DCM gelöst und bei 0°C mit Methansulfonsäure (60 mL, 933 mmol) und Polyphosphorsäure (91 g, 933 mmol) versetzt. Man lässt die Reaktionslösung über Nacht auf Raumtemperatur erwärmen. Die Mischung wird mit Ethanol erweitert und eingeengt. Der Rückstand wird in Toluol gelöst, mit NaOH-Lösung und dest. Wasser gewaschen und über Magnesiumsulfat getrocknet. Nach Entfernen des Lösemittels im Vakuum wird der Feststoff in Ethanol umkristallisiert. Man isoliert 55 g eines gelben Feststoffes. (93% d. Th.).

Analog dazu werden folgende Verbindungen hergestellt:

| Edukt 1 | Produkt | Ausbeute (4 Stufe) |
|---|---|---|
| 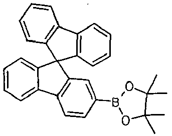 | 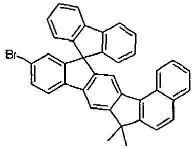 | 68% |
| 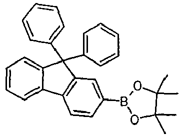 | 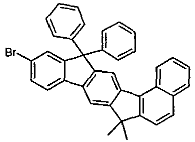 | 59% |
| 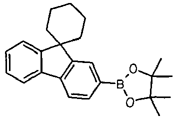 | 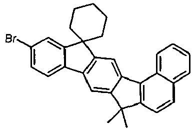 | 64% |
| 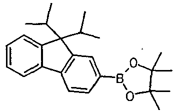 | 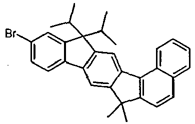 | 74% |

### III-2) Synthese der Bausteine (ii-1)

Allgemeines Reaktionsschema:

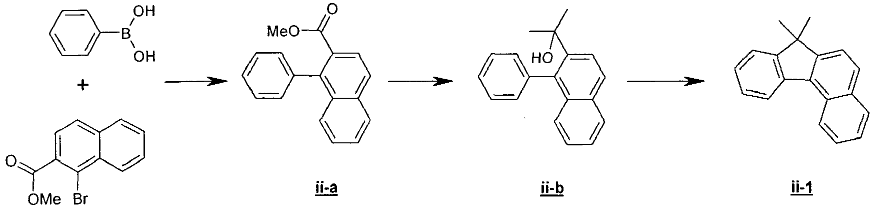

### 1-Phenyl-naphthalen-2-carboxylsäuremethylester (ii-a)

1-Brom-naphthalen-2-carboxylsäuremethylester (70.0 g, 264 mmol), Phenylboronsäure (38.6 g, 317 mmol) und Kaliumphosphatmonohydrat (182 g, 792 mmol) werden in einer Mischung von 0.2 L Toluol, 0.2 L Dioxan und 0.2 L Wasser gelöst und mit Palladiumacetat (1.18 g, 5.3 mmol) und Tri-orthotolyl-Phosphin (3.2 g, 10.6 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über Aluminiumoxid filtriert. Die organische Phase wird bis zu einem orangen Öl eingeengt. Es werden 69 g Produkt erhalten (99% d. Th.).

### 2-(1-Phenyl-naphthalen-2-yl)-propan-2-ol (ii-b)

**(ii-a)** (69 g, 264 mmol) und Cerium(III) Chlorid (71 g, 291 mmol) werden in 500 mL THF gelöst und bei 0°C mit Methylmagnesiumchlorid (3 M Lösung in THF) (308 mL, 925 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels im Vakuum erhält man einen hellbeigen Feststoff. Dieser wird in einem Heptan/ Toluol-Gemisch umkristallisiert. Man isoliert 52 g (75% d. Th.) als farblosen Feststoff.

### 7,7-Dimethyl-7H-benzo[c]fluorene (ii-1)

**(ii-b)** (52 g, 198 mmol) wird mit 500 mL DCM gelöst und bei 0°C mit Methansulfonsäure (64 mL, 991 mmol) und Polyphosphorsäure (77 g, 793 mmol) versetzt. Man lässt die Reaktionslösung über Nacht auf Raumtemperatur erwärmen. Die Mischung wird mit Ethanol erweitert und eingeengt. Der Rückstand wird in Toluol gelöst, mit NaOH-Lösung und dest. Wasser gewaschen und über Magnesiumsulfat getrocknet. Nach Entfernen des Lösemittels im Vakuum wird der Feststoff in Ethanol umkristallisiert. Man isoliert 44 g eines gelben Feststoffes. (91% d. Th.).

### III-3) Synthese des Bausteins (ii-2)

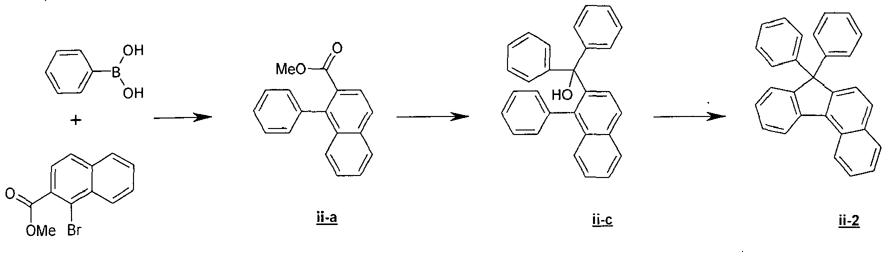

### Diphenyl-(1-Phenyl-naphthalen-2-yl)-methanol (ii-c)

(ii-a) (35 g, 133 mmol) und Cerium(III) Chlorid (36 g, 146 mmol) werden in 250 mL THF gelöst und bei 0°C mit Phenylmagnesiumchlorid (3 M Lösung in THF) (150 mL, 450 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels im Vakuum erhält man einen hellbeigen Feststoff. Dieser wird in Heptan/Toluol umkristallisiert. Man isoliert 41 g (80% d. Th.) als farblosen Feststoff.

Der Baustein **(ii-2)** wird analog zu **(ii-1)** synthetisiert, mit einer Ausbeute von 88%.

### III-4) Synthese des Bausteins (ii-3)

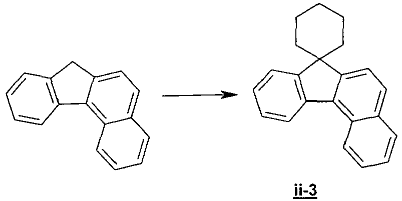

### 7H-Benzo[c]fluoren wurde gemäß folgender Literaturvorschrift synthetisiert: Organic Letters, 2009, Vol. 11, No. 20, 4588-4591.

### Synthese von (ii-3)

7H-Benzo[c]fluoren (38 g, 176 mmol), 1,5-Dibrompentan (40.5 g, 176 mmol) und Tetrabutylammoniumbromid (32.3 g, 100 mmol) werden in 0.5 L Toluol gelöst. 0.5 L 3M NaOH Lösung wird zugegeben, und die Reaktion wird über Nacht auf Rückfluss gekocht. Die Reaktion wird auf Raumtemperatur abgekühlt, die Phasen getrennt, die wässrige Phase wird mit Toluol drei Mal extrahiert. Die organische Phase wird mit dest. Wasser gewaschen, getrocknet und vom Lösungsmittel befreit. Der erhaltene Feststoff wird in Toluol Heptan umkristallisiert. 31 g eines farblosen Feststoffes (62% d. Th.) werden isoliert.

### III-5) Synthese des Bausteins (iii)

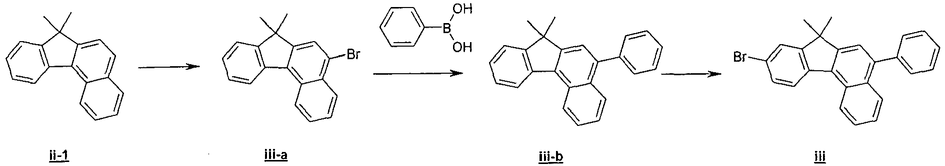

### 5-Bromo-7,7-dimethyl-7H-benzo[c]fluorene (iii-a)

**(ii-1)** (38.2 g, 156 mmol) wird in 0.3 L Chloroform gelöst und auf 0°C gekühlt. Unter Rühren wird eine Dibrom-Lösung (117 g, 660 mmol) in 0.2 L Chloroform zugetropft, so dass die Reaktionstemperatur nicht über 5 °C steigt. Die Reaktion wird über Nacht im Eisbad auf Raumtemperatur erwärmt. 200 mL einer 10%igen Natriumthiosulfat-Lösung werden hinzu gegeben und die Phasen getrennt. Die wässrige Phase wird mit DCM mehrmals extrahiert. Die organische Phase wird mit dest. Wasser gewaschen, getrocknet und vom Lösungsmittel befreit. Der erhaltene Feststoff wird in Toluol/Heptan umkristallisiert. Man erhält 50 g eines farblosen Feststoffes (99% d. Th.).

### 7,7-Dimethyl-5-Phenyl-7H-benzo[c]fluoren (iii-b)

(iii-a) (28.5 g, 88 mmol), Phenylboronsäure (13.2 g, 106 mmol) und Kaliumcarbonat (30.5 g, 220 mmol) werden in einer Mischung von 150 mL Toluol und 150 mL Wasser gelöst und mit Tetrakis-(triphenylphosphin)-palladium (1.02 g, 0.9 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über AlOx und Kieselgel filtriert. Die organische Phase wird eingeengt und der resultierende Feststoff mit Ethanol gewaschen. Es werden 25.9 g (92% d. Th.) Produkt erhalten.

### 9-Bromo-7,7-dimethyl-5-Phenyl-7H-benzo[c]fluoren (iii)

**(iii-b)** (25.8 g, 81 mmol) wird in 0.15 L Chloroform gelöst und auf 0 °C gekühlt. Unter Rühren wird eine Dibrom-Lösung (13.6 g, 85 mmol) in 0.1 L Chloroform zugetropft, so dass die Reaktionstemperatur nicht über 5 °C steigt. Die Reaktion wird über Nacht im Eisbad auf Raumtemperatur erwärmt. 100 mL einer 10%igen Natriumthiosulfat-Lösung werden hinzu gegeben und die Phasen getrennt. Die wässrige Phase wird mit DCM mehrmals extrahiert. Die organische Phase wird mit dest. Wasser gewaschen, getrocknet und vom Lösungsmittel befreit. Der erhaltene Feststoff wird in Toluol/Heptan umkristallisiert. Es werden 22 g eines farblosen Feststoffs (62% d. Th.) erhalten.

Analog dazu werden folgende Verbindungen hergestellt:

| Edukt ii oder Analog | Boronsäure Ar-B(OH)₂ | Produkt (iii Analog) | Ausbeute (3 Stufe) |
|---|---|---|---|
| 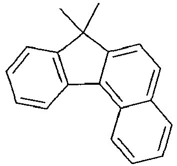 | 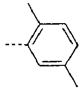 | 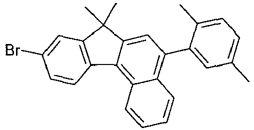 | 66% |
| 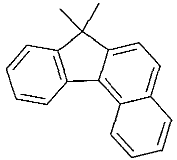 | 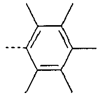 | 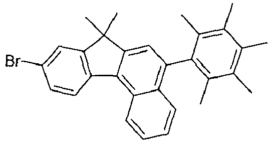 | 65% |
| 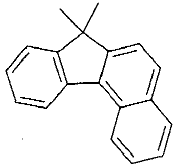 | 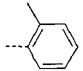 | 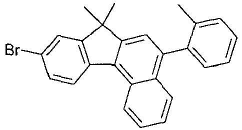 | 70% |
| 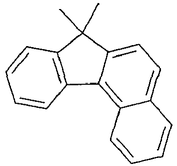 | 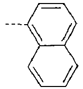 | 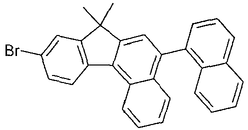 | 61% |
| 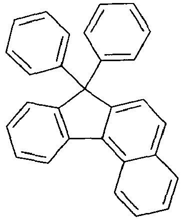 | 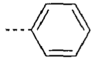 | 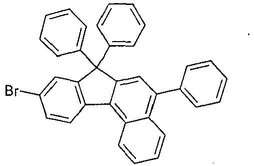 | 53% |
| 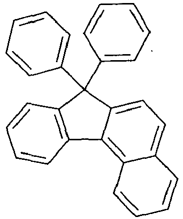 | 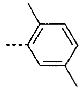 | 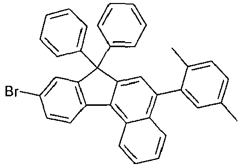 | 61% |
| 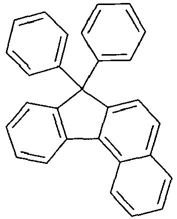 | 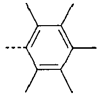 | 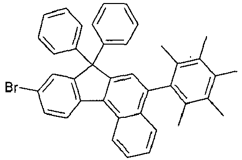 | 37% |
| 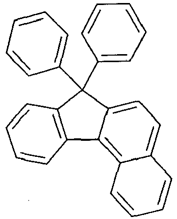 | 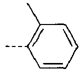 | 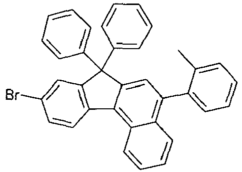 | 64% |
| 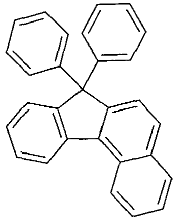 | 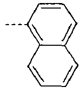 | 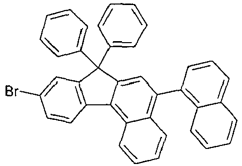 | 31% |
| 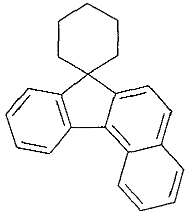 | 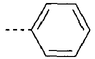 | 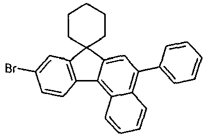 | 50% |
| 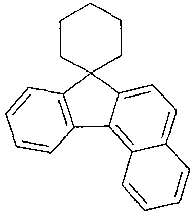 | 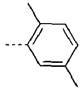 | 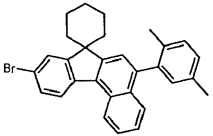 | 54% |
| 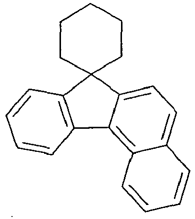 | 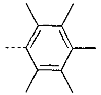 | 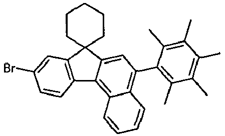 | 34% |
| 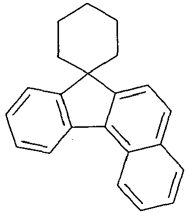 | 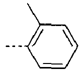 | 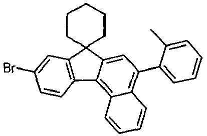 | 58% |

### III-6) Synthese der Bausteine (iv)

Allgemeines Reaktionsschema:

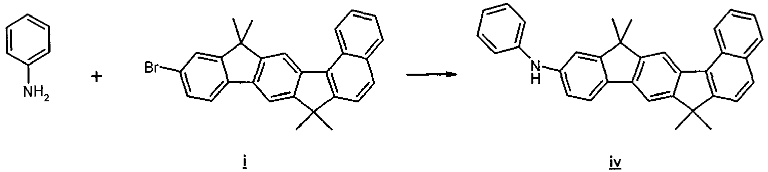

### N-Phenyl.7,7,13,13-tetramethyl-7,13-dihydrobenzo[g]indeno[1,2-b]fluoren-11-amin (iv)

i (37 g, 84.2 mmol) und Anilin (8.6 g, 92.6 mmol) werden in 500 mL Toluol gelöst. Die Lösung wird entgast und mit Argon gesättigt. Danach wird sie mit 4.1 g (5.1 mmol) Pd(dppf)Cl₂ versetzt. Anschließend werden 24.3 g Natrium-*tert*.-butylat (253 mmol) zugegeben. Die Reaktionsmischung wird für 12h unter Schutzgasatmosphäre zum Sieden erhitzt. Das Gemisch wird im Anschluss mit Wasser versetzt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 31 g (77 % d. Th).

| Edukt **i** oder Analog | Amin | Produkt **iv** Analog | Ausbeute |
|---|---|---|---|
| 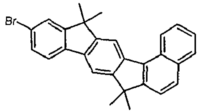 | 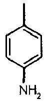 | 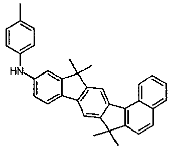 | 58% |
| 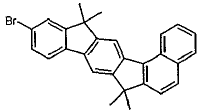 | 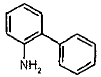 | 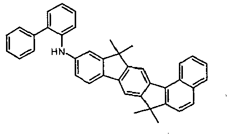 | 72% |
| 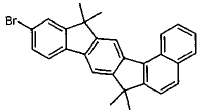 | 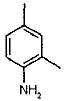 | 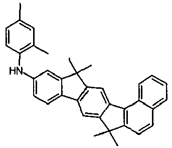 | 62% |
| 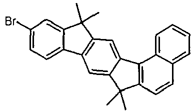 | 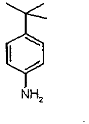 | 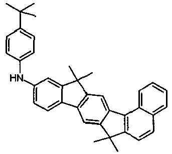 | 51% |
| 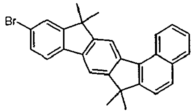 | 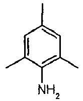 | 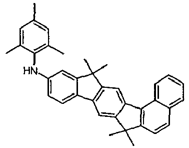 | 63% |
| 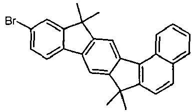 | 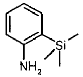 | 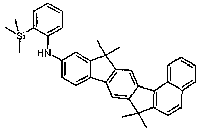 | 39% |
| 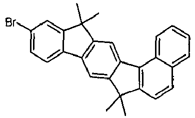 | 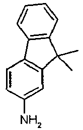 | 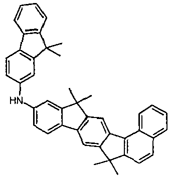 | 57% |
| 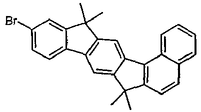 | 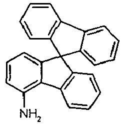 | 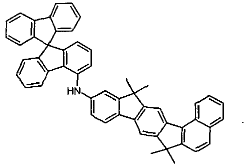 | 59% |
| 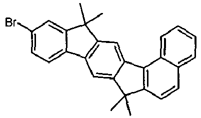 | 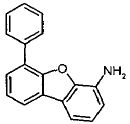 | 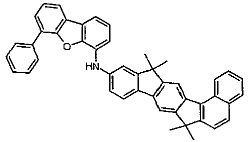 | 64% |
| 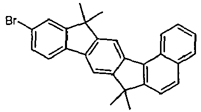 | 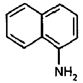 | 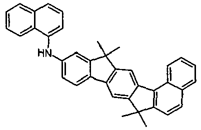 | 32% |
| 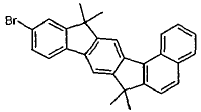 | 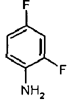 | 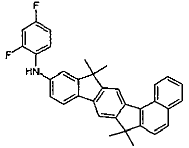 | 61% |
| 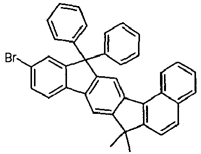 | 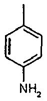 | 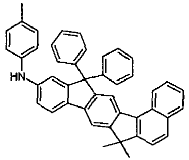 | 64% |
| 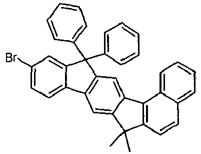 | 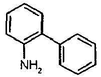 | 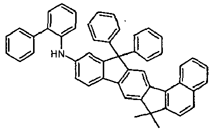 | 68% |
| 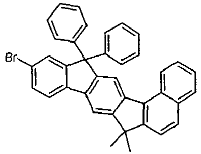 | 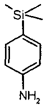 | 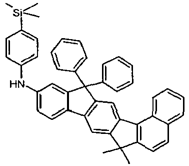 | 39% |
| 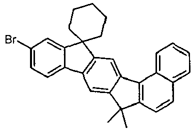 | 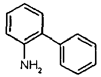 | 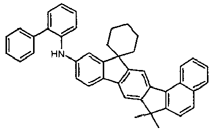 | 65% |
| 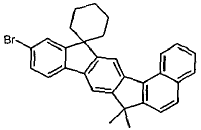 | 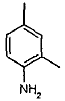 | 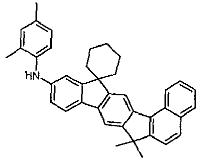 | 58% |
| 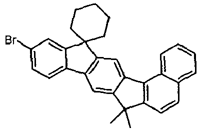 | 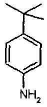 | 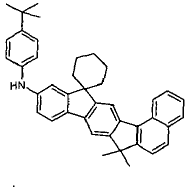 | 52% |
| 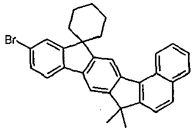 | 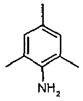 | 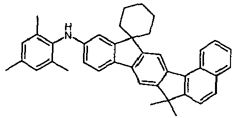 | 66% |
| 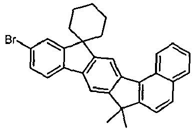 | 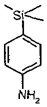 | 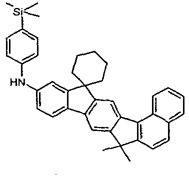 | 46% |
| 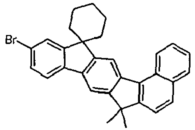 | 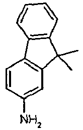 | 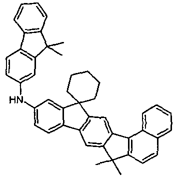 | 72% |
| 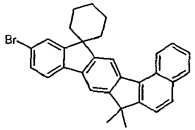 | 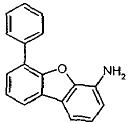 | 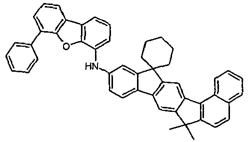 | 64% |
| 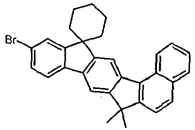 | 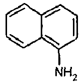 | 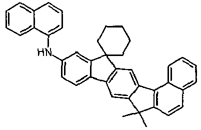 | 33% |
| 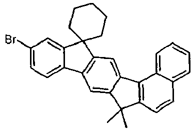 | 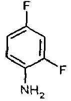 | 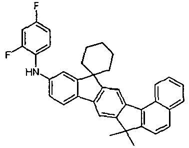 | 54% |
| 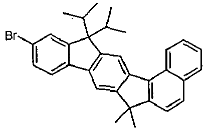 | 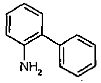 | 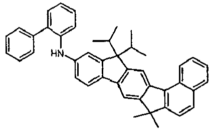 | 47% |
| 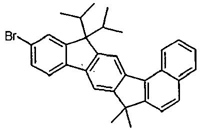 | 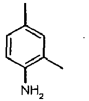 | 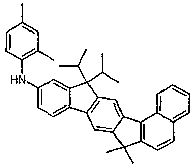 | 41% |

### III-7) Synthese der Zielverbindungen (III)

Allgemeines Reaktionsschema:

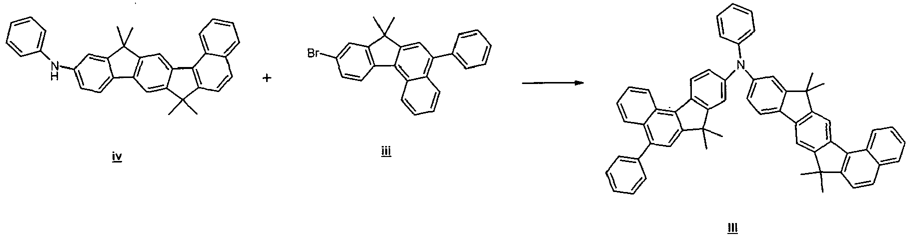

### 7,7,13,13-Tetramethyl-N-(7,7-dimethyl-5-phenyl-7H-benzo[c]fluoren-9-yl)-N-phenyl-7,13-dihydrobenzo[g]indeno[1,2-b]fluoren-11-amin (III) (Synthesebeispiel 37)

**iv** (20 g, 44.3 mmol) und **iii** (18.6 g, 46.5 mmol) werden in 500 mL Toluol gelöst. Die Lösung wird entgast und mit Argon gesättigt. Danach wird sie mit 2.5 mL (2.5 mmol) einer 1M Tri-*tert*.-Butylphosphin Lösung und 0.355 g (1.23 mmol) Palladium(II)-acetat versetzt. Anschließend werden 11.9 g Natrium-tert.-butylat (124 mmol) zugegeben. Die Reaktionsmischung wird für 12h unter Schutzgasatmosphäre zum Sieden erhitzt. Das Gemisch wird im Anschluss mit Wasser versetzt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 21 g (62 % d. Th).

Analog dazu werden folgende Verbindungen hergestellt:

| Bsp. | Edukt **iv** oder Analog | Edukt **iii** oder Analog | Produkt **III** Analog | Ausbeute |
|---|---|---|---|---|
| 38 | 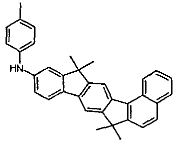 | 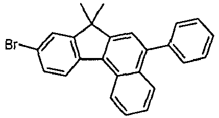 | 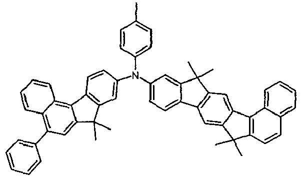 | 74% |
| 39 | 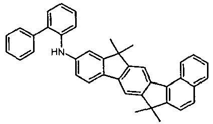 | 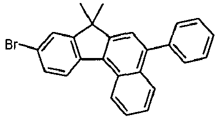 | 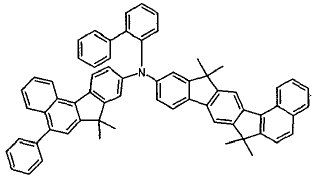 | 81% |
| 40 | 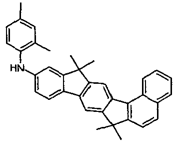 | 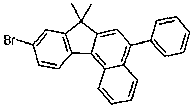 | 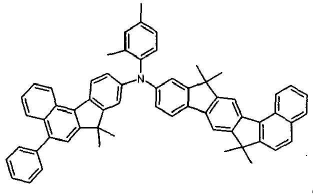 | 69% |
| 41 | 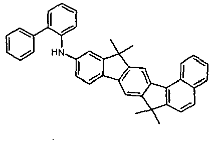 | 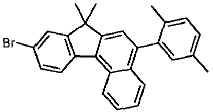 | 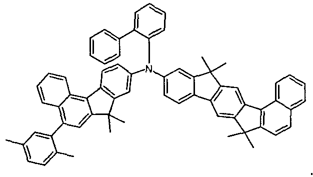 | 75% |
| 42 | 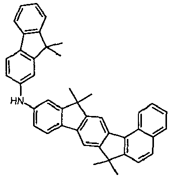 | 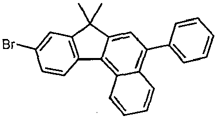 | 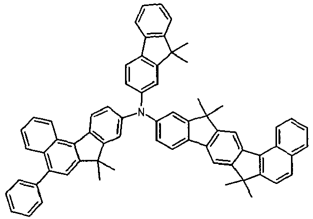 | 52% |
| 43 | 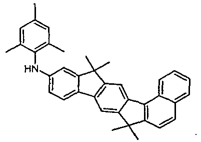 | 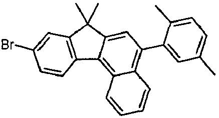 | 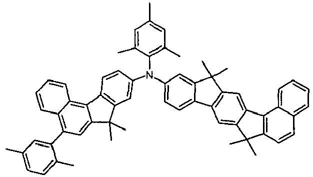 | 58% |
| 44 | 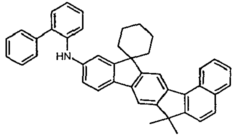 | 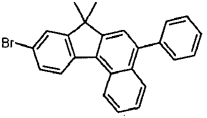 | 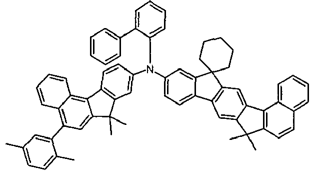 | 33% |
| 45 | 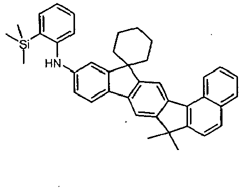 | 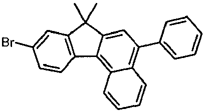 | 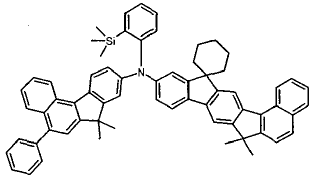 | 79% |
| 46 | 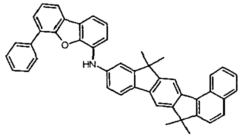 | 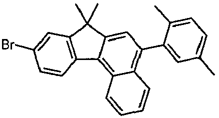 | 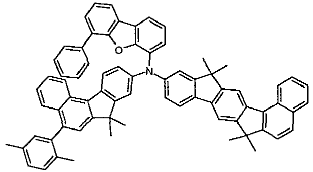 | 54% |
| 47 | 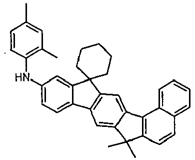 | 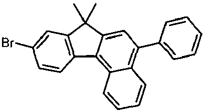 | 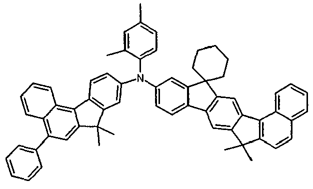 | 81% |
| 48 | 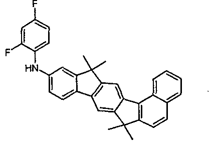 | 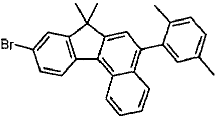 | 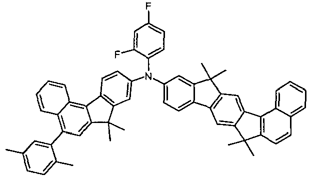 | 44% |
| 49 | 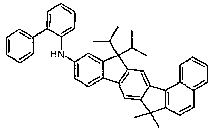 | 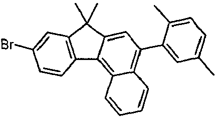 | 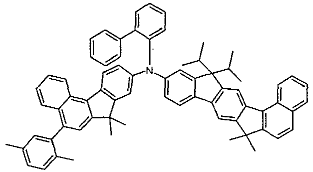 | 63% |
| 50 | 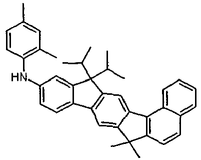 | 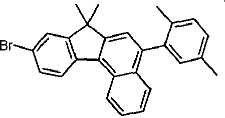 | 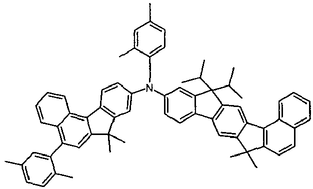 | 71% |

### B) Devicebeispiele: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 bis 3) werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Buffer (20nm) / Lochinjektionsschicht (HIL, 5nm) / Lochtransportschicht (HTL, 30nm) / Emissionsschicht (EML, 20nm) / Elektronentransportschicht (ETL, 30nm) / Elektroneninjektionsschicht (LiQ 1nm) und abschließend eine Kathode. Die Kathode wird durch eine 100 nm dicke Aluminiumschicht gebildet. Als Buffer wird eine 20nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft. Der Aufbau von EML und ETL der OLEDs ist in Tabelle 1 gezeigt. Die verwendeten Materialien sind in Tabelle 3 gezeigt.

Die Emissionsschicht (EML) besteht immer aus mindestens einem Matrixmaterial (Host=H) und einem emittierenden Dotierstoff (Dotand=D), der dem Matrixmaterial durch Coverdampfung in einem bestimmten Volumenanteil beigemischt wird. Eine Angabe wie H1:D1 (95%:5%) bedeutet hierbei, dass das Material H1 in einem Volumenanteil von 95% und D1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die Lebensdauer LD50 @ 60mA/cm² ist die Zeit, die vergeht, bis die Starthelligkeit (cd/m²) bei einer Stromdichte von 60mA/cm² auf die Hälfte gesunken ist. Die erhaltenen Daten für die verschiedenen OLEDs sind in Tabelle 2 zusammengefasst.

### Verwendung von erfindungsgemäßen Verbindungen als Dotanden in fluoreszierenden OLEDs

Insbesondere eignen sich erfindungsgemäße Verbindungen als blaue fluoreszierende Dotanden. Als Vergleichs-Dotanden werden die im Stand der Technik bekannten Dotanden V-D1 und V-D2 (WO 2006/108497 und WO 2008/006449) verwendet. Als erfindungsgemäße Beispiele werden die Dotanden D3, D4, D5, D6 und D7 vermessen.

| **Tabelle 1: Aufbau der OLEDs** | | |
|---|---|---|
| ***Bsp.*** | ***EML*** | ***ETL*** |
| | *Dicke* / *nm* | *Dicke* / *nm* |
| *V1* | *H1(95%):V-D1(5%) 20 nm* | *ETM1(50%):LiQ(50%)30 nm* |
| *V2* | *H3(95%): V-D1(5%)20 nm* | *ETM1(5O%):LiQ(50%)30 nm* |
| *V3* | *H1(95%):V-D2(5%)20 nm* | *ETM1(50%):LiQ(5O%)30 nm* |
| *V4* | *H3(95%):V-D2(5%)20 nm* | *ETM1 (50%):LiQ(50%)30 nm* |
| *E5* | *H1(95%):D3(5%) 20 nm* | *ETM1(50%):LiQ(50%)30 nm* |
| *E6* | *H2(95%)D3(5%)20 nm* | *ETM1(50%):LiQ(50%)30 nm* |
| *E7* | *H1 (95%):D4(5%)20 nm* | *ETM1(50%):LiQ(50%)30 nm* |
| *E8* | *H3(95%):D4(5%)20 nm* | *ETM1(50%):LiQ(50%)30 nm* |
| *E9* | *H1(95%):D5(5%) 20 nm* | *ETM1(50%):LiQ(50%)30 nm* |
| *E10* | *H2(95%)D5(5%)20 nm* | *ETM1(50%):LiQ(50%)30 nm* |
| *E11* | *H1(95%):D6(5%)20 nm* | *ETM1(50%):LiQ(50%)30 nm* |
| *E12* | *H3(95%):VD6(5%) 20 nm* | *ETM1(50%):LiQ(50%)30 nm* |
| *E13* | *H1(95%):D7(5%) 20 nm* | *ETM1(50%):LiQ(50%)30 nm* |

| **Tabelle 2: Daten der OLEDs** | | | | |
|---|---|---|---|---|
| ***Bsp.*** | ***EQE* @ *1000 cd*/*m2*** | ***LD50* @ *60mA*/*cm²*** | ***CIE*** | |
| | % | *[h]* | *x* | *v* |
| *V1* | *2*.*4* | *110* | 0.16 | 0.09 |
| *V2* | *2.3* | *120* | 0.16 | 0.10 |
| *V3* | *2.6* | *260* | 0.15 | 0.17 |
| *V4* | *2.5* | *280* | 0.15 | 0.18 |
| *E5* | *6.2* | *560* | 0.14 | 0.10 |
| *E6* | *6.4* | *620* | 0.14 | 0.11 |
| *E7* | *6.9* | *580* | *0.14* | *0.12* |
| *E8* | *7.1* | *600* | *0.14* | *0.13* |
| *E9* | *6.5* | *560* | *0.13* | *0.10* |
| *E10* | *6.8* | *610* | *0.13* | *0.11* |
| *E11* | *5.2* | *450* | *0.13* | *0.08* |
| *E12* | *5.6* | *510* | *0.14* | *0.09* |
| *E13* | *6.7* | *420* | *0.14* | *0.08* |

| **Tabelle 3: Strukturen der verwendeten Materialien** | |
|---|---|
| 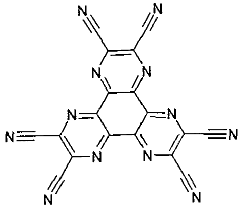 | 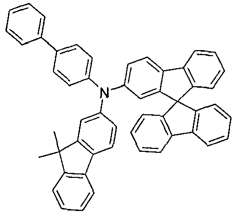 |
| HIL1 | HTL |
| 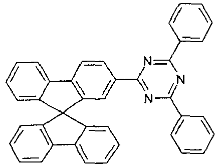 | 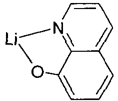 |
| ETM1 | LiQ |
| 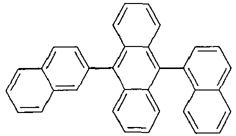 | 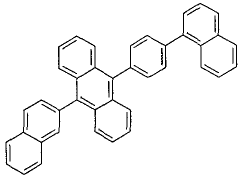 |
| H1 | H2 |
| 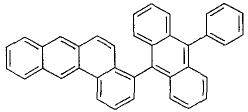 | 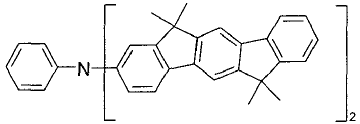 |
| H3 | V-D1 |
| 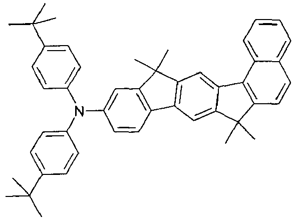 | 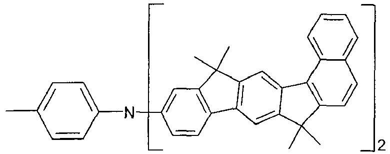 |
| V-D2 | D3 |
| 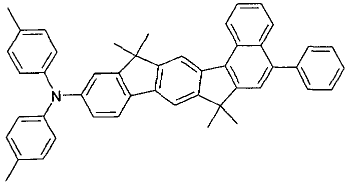 | 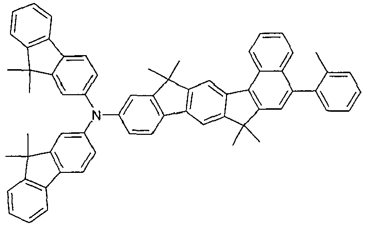 |
| D4 | D5 |
| 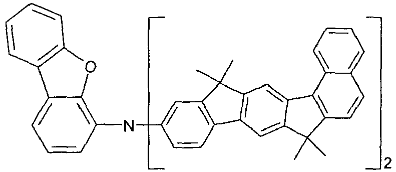 | 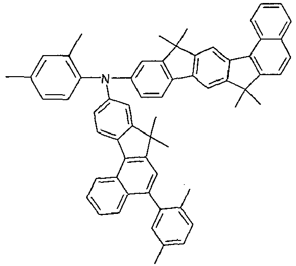 |
| D6 | D7 |

Die Ergebnisse zeigen, dass mit den erfindungsgemäßen Verbindungen effiziente OLEDs (externe Quanteneffizienz) mit hoher Lebensdauer (LD50) erhalten werden können, bei tiefblauer Emission.

Im Vergleich dazu zeigen die im Stand der Technik bekannten Dotanden V-D1 und V-D2 deutliche schlechtere Werte für die Effizienz und die Lebensdauer.

## Patentansprüche

1. Verbindung der Formel (I), **(II)** oder (III) 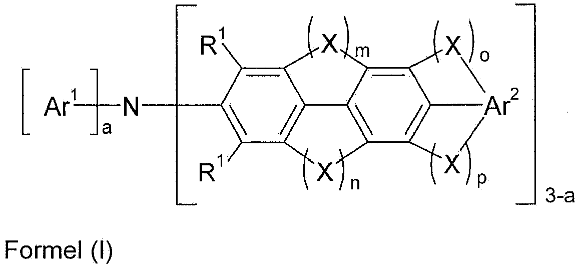 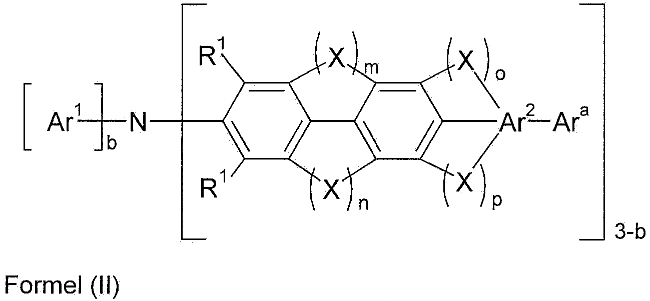 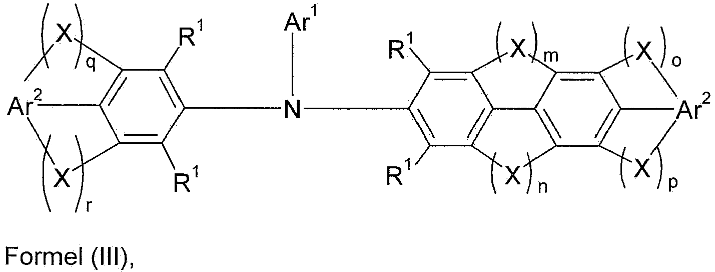 wobei gilt:
Ar¹ ist bei jedem Auftreten gleich oder verschieden gewählt aus einem aromatischen oder heteroaromatischen Ringsystem mit 5 bis 30 aromatischen Ringatomen, welches mit einem oder mehreren Resten R¹ substituiert sein kann;
Ar² ist bei jedem Auftreten gleich oder verschieden eine Aryl- oder Heteroarylgruppe mit 10 bis 30 aromatischen Ringatomen, welche mit einem oder mehreren Resten R¹ substituiert sein kann;
Ar^{a} ist bei jedem Auftreten gleich oder verschieden eine Aryl- oder Heteroarylgruppe mit 5 bis 30 aromatischen Ringatomen, welche mit einem oder mehreren Resten R¹ substituiert sein kann;
R¹ ist bei jedem Auftreten gleich oder verschieden H, D, F, Cl, Br, I, C(=O)R², CN, Si(R²)3, N(R²)2, NO₂, P(=O)(R²)₂, S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, oder eine Aryloxy- oder Heteroaryloxygruppe mit 5 bis 30 aromatischen Ring-atomen, die durch einen oder mehrere Reste R² substituiert sein kann, wobei zwei Reste R¹, welche an dieselbe Gruppe X gebunden sind, miteinander einen Ring bilden können;
R² ist bei jedem Auftreten gleich oder verschieden H, D, F, Cl, Br, I, C(=O)R³, CN, Si(R³)₃, N(R³)₂, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³⁻, -C≡C-, Si(R³)₂, C=O, C=S, C=NR³, -C(=O)O-, -C(=O)NR³-, NR³, P(=O)(R³), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ring-atomen, die durch einen oder mehrere Reste R³ substituiert sein kann;
R³ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
X ist bei jedem Auftreten gleich oder verschieden C(R¹)₂ oder Si(R¹)₂;
a ist gleich 0 oder 1;
b ist gleich 0, 1 oder 2;
m, n, o, p, q und r sind bei jedem Auftreten gleich oder verschieden 0 oder 1; wobei im Fall, dass sie 0 sind, an den betreffenden Positionen, an die die entsprechende Gruppe X bindet, stattdessen eine Gruppe R¹ gebunden ist;
wobei die Summe von m und n gleich 1 ist, und die Summe von o und p gleich 1 ist, und die Summe von q und r gleich 1 oder 2 ist.

2. Verbindung nach Anspruch 1, **dadurch gekennzeichnet, dass** Ar¹ bei jedem Auftreten gleich oder verschieden gewählt ist aus Phenyl, Naphthyl, Anthracenyl, Phenanthrenyl, Pyrenyl, Triphenylenyl, Chrysenyl, Biphenyl, Terphenyl, Fluorenyl, Spirobifluorenyl, Carbazolyl, Dibenzofuranyl, Dibenzothiophenyl oder Silafluorenyl, das jeweils mit einem oder mehreren Resten R¹ substituiert sein kann.

3. Verbindung nach Anspruch 1 oder 2, **dadurch gekennzeichnet, dass** Ar² gewählt ist aus den folgenden Gruppen der Formeln (Ar²-a) bis (Ar²-h):
| | |
|---|---|
| 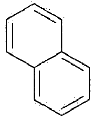 | 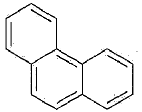 |
| Formel (Ar²-a) | Formel (Ar²-b) |
| 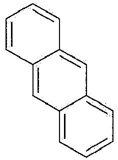 | 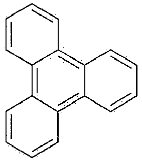 |
| Formel (Ar²-c) | Formel (Ar²-d) |
| 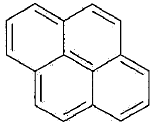 | 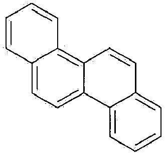 |
| Formel (Ar²-e) | Formel (Ar²-f) |
| 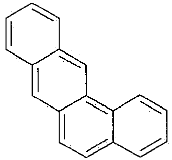 | 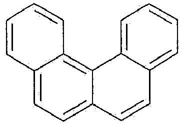 |
| Formel (Ar²-g) | Formel (Ar²-h) |
wobei die Anbindungspositionen an den Rest der Formel an beliebigen Positionen vorliegen können und wobei die Gruppen mit einem oder mehreren Resten R¹ substituiert sein können.

4. Verbindung nach einem oder mehreren der Ansprüche 1 bis 3, **dadurch gekennzeichnet, dass** Ar^{a} eine Arylgruppe mit 6 bis 18 aromatischen Ringatomen ist, die mit einem oder mehreren Resten R¹ substituiert sein kann.

5. Verbindung der Formel (III) nach einem oder mehreren der Ansprüche 1 bis 4, **dadurch gekennzeichnet, dass** Ar² mit einer oder mehreren Gruppen R¹ substituiert ist, die gewählt sind aus einem aromatischen oder heteroaromatischen Ringsystem mit 5 bis 30 aromatischen Ringatomen, das mit einem oder mehreren Resten R² substituiert sein kann.

6. Verbindung nach einem oder mehreren der Ansprüche 1 bis 5, **dadurch gekennzeichnet, dass** R¹ bei jedem Auftreten gleich oder verschieden gewählt ist aus H, D, F, CN, Si(R²)3, einer geradkettigen Alkylgruppe mit 1 bis 8 C-Atomen oder einer verzweigten oder cyclischen Alkylgruppe mit 3 bis 8 C-Atomen, wobei die Alkylgruppen jeweils mit einem oder mehreren Resten R² substituiert sein können und wobei in den Alkylgruppen eine oder mehrere CH₂-Gruppen durch -C=C-, -R²C=CR²-, Si(R²)₂, C=O oder -O- ersetzt sein können, oder einer Aryl- oder Heteroarylgruppe mit 6 bis 16 aromatischen Ringatomen, die jeweils mit einem oder mehreren Resten R² substituiert sein kann.

7. Verbindung nach einem oder mehreren der Ansprüche 1 bis 6, **dadurch gekennzeichnet, dass** a gleich 1 ist.

8. Verbindung nach einem oder mehreren der Ansprüche 1 bis 7, **dadurch gekennzeichnet, dass** b gleich 2 ist.

9. Verbindung nach einem oder mehreren der Ansprüche 1 bis 8, **dadurch gekennzeichnet, dass** die Summe aus q und r gleich 1 ist.

10. Oligomere, Polymere oder Dendrimere enthaltend eine oder mehrere Verbindungen nach einem oder mehreren der Ansprüche 1 bis 9, wobei die Bindung(en) zum Polymer, Oligomer oder Dendrimer an beliebigen, in Formel (I), (II) oder (III) mit R¹ oder R² substituierten Positionen lokalisiert sein können.

11. Formulierung, enthaltend mindestens eine Verbindung nach einem oder mehreren der Ansprüche 1 bis 9 oder mindestens ein Polymer, Oligomer oder Dendrimer nach Anspruch 10 sowie mindestens ein Lösungsmittel.

12. Elektronische Vorrichtung, gewählt aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs), **dadurch gekennzeichnet, dass** sie mindestens eine Verbindung nach einem oder mehreren der Ansprüche 1 bis 9 oder mindestens ein Polymer, Oligomer oder Dendrimer nach Anspruch 10 enthält.

13. Elektronische Vorrichtung nach Anspruch 12, gewählt aus organischen Elektrolumineszenzvorrichtungen, **dadurch gekennzeichnet, dass** die Verbindung nach einem oder mehreren der Ansprüche 1 bis 9 oder das Polymer, Oligomer oder Dendrimer nach Anspruch 10 als emittierendes Material in einer emittierenden Schicht eingesetzt wird.

14. Verfahren zur Herstellung einer Verbindung nach einem oder mehreren der Ansprüche 1 bis 9, **dadurch gekennzeichnet, dass** eine oder mehrere metallorganische Kupplungsverfahren eingesetzt werden.

## Claims

1. Compound of the formula (I), (II) or (III) 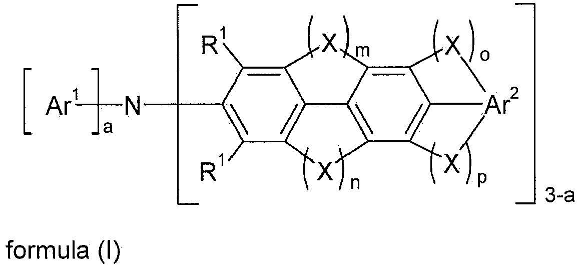 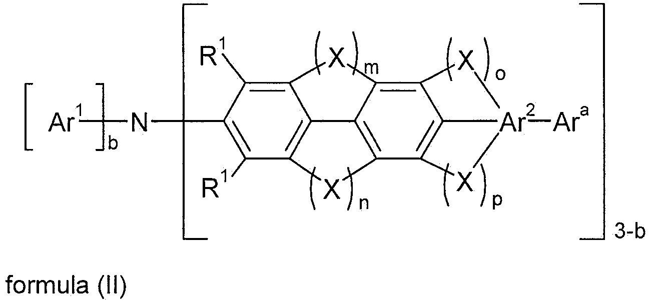 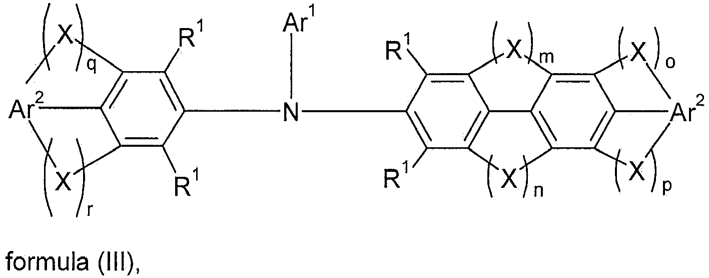 where:
Ar¹ is selected on each occurrence, identically or differently, from an aromatic or heteroaromatic ring system having 5 to 30 aromatic ring atoms, which may be substituted by one or more radicals R¹;
Ar² is on each occurrence, identically or differently, an aryl or heteroaryl group having 10 to 30 aromatic ring atoms, which may be substituted by one or more radicals R¹;
Ar^{a} is on each occurrence, identically or differently, an aryl or heteroaryl group having 5 to 30 aromatic ring atoms, which may be substituted by one or more radicals R¹;
R¹ is on each occurrence, identically or differently, H, D, F, Cl, Br, I, C(=O)R², CN, Si(R²)3, N(R²)2, NO₂, P(=O)(R²)2, S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the abovementioned groups may in each case be substituted by one or more radicals R² and where one or more CH₂ groups in the above-mentioned groups may be replaced by -R²C=CR²-, -C≡C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, l, CN or NO₂, or an aromatic or heteroaromatic ring system having 5 to 30 aromatic ring atoms, which may in each case be substituted by one or more radicals R², or an aryloxy or heteroaryloxy group having 5 to 30 aromatic ring atoms, which may be substituted by one or more radicals R², where two radicals R¹ which are bonded to the same group X may form a ring with one another;
R² is on each occurrence, identically or differently, H, D, F, Cl, Br, I, C(=O)R³, CN, Si(R³)3, N(R³)2, NO₂, P(=O)(R³)2, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the abovementioned groups may in each case be substituted by one or more radicals R³ and where one or more CH₂ groups in the above-mentioned groups may be replaced by -R³C=CR³-, -C≡C-, Si(R³)2, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³;
R³ is on each occurrence, identically or differently, H, D, F or an aliphatic, aromatic or heteroaromatic organic radical having 1 to 20 C atoms, in which, in addition, one or more H atoms may be replaced by D or F;
X is on each occurrence, identically or differently, C(R¹)₂ or Si(R¹)₂;
a is equal to 0 or 1;
b is equal to 0, 1 or 2;
m, n, o, p, q and r are on each occurrence, identically or differently, 0 or 1; where, in the case where they are 0, a group R¹ is bonded instead at the relevant positions to which the corresponding group X is bonded;
where the sum of m and n is equal to 1 or 2, and the sum of o and p is equal to 1 or 2, and the sum of q and r is equal to 1 or 2.

2. Compound according to Claim 1, **characterised in that** Ar¹ is selected on each occurrence, identically or differently, from phenyl, naphthyl, anthracenyl, phenanthrenyl, pyrenyl, triphenylenyl, chrysenyl, biphenyl, terphenyl, fluorenyl, spirobifluorenyl, carbazolyl, dibenzo-furanyl, dibenzothiophenyl or silafluorenyl, which may in each be substituted by one or more radicals R¹.

3. Compound according to Claim 1 or 2, **characterised in that** Ar² is selected from the following groups of the formulae (Ar²-a) to (Ar²-h):
| | |
|---|---|
| 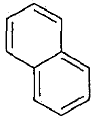 | 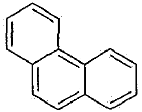 |
| formula (Ar²-a) | formula (Ar²-b) |
| 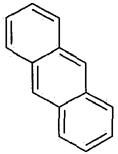 | 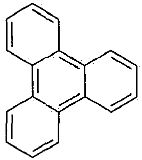 |
| formula (Ar²-c) | formula (Ar²-d) |
| 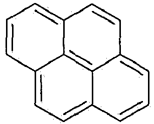 | 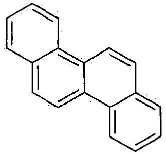 |
| formula (Ar²-e) | formula (Ar²-f) |
| 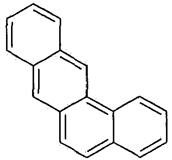 | 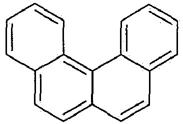 |
| formula (Ar²-q) | formula (Ar²-h) |
where the bonding positions to the radical of the formula may be at any desired positions and where the groups may be substituted by one or more radicals R¹.

4. Compound according to one or more of Claims 1 to 3, **characterised in that** Ar^{a} is an aryl group having 6 to 18 aromatic ring atoms, which may be substituted by one or more radicals R¹.

5. Compound of the formula (III) according to one or more of Claims 1 to 4, **characterised in that** Ar² is substituted by one or more groups R¹, which are selected from an aromatic or heteroaromatic ring system having 5 to 30 aromatic ring atoms, which may be substituted by one or more radicals R².

6. Compound according to one or more of Claims 1 to 5, **characterised in that** R¹ is selected on each occurrence, identically or differently, from H, D, F, CN, Si(R²)₃, a straight-chain alkyl group having 1 to 8 C atoms or a branched or cyclic alkyl group having 3 to 8 C atoms, where the alkyl groups may in each case be substituted by one or more radicals R² and where one or more CH₂ groups in the alkyl groups may be replaced by -C≡C-, -R²C=CR²-, Si(R²)₂, C=O or -O-, or an aryl or heteroaryl group having 6 to 16 aromatic ring atoms, which may in each case be substituted by one or more radicals R².

7. Compound according to one or more of Claims 1 to 6, **characterised in that** a is equal to 1.

8. Compound according to one or more of Claims 1 to 7, **characterised in that** b is equal to 2.

9. Compound according to one or more of Claims 1 to 8, **characterised in that** the sum of q and r is equal to 1.

10. Oligomers, polymers or dendrimers containing one or more compounds according to one or more of Claims 1 to 9, where the bond(s) to the polymer, oligomer or dendrimer may be localised at any desired positions in formula (I), (II) or (III) that are substituted by R¹ or R².

11. Formulation comprising at least one compound according to one or more of Claims 1 to 9 or at least one polymer, oligomer or dendrimer according to Claim 10 and at least one solvent.

12. Electronic device, selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs), **characterised in that** it contains at least one compound according to one or more of Claims 1 to 9 or at least one polymer, oligomer or dendrimer according to Claim 10.

13. Electronic device according to Claim 12, selected from organic electroluminescent devices, **characterised in that** the compound according to one or more of Claims 1 to 9 or the polymer, oligomer or dendrimer according to Claim 10 is employed as emitting material in an emitting layer.

14. Process for the preparation of a compound according to one or more of Claims 1 to 9, **characterised in that** one or more organometallic coupling processes are employed.

## Revendications

1. Composé de la formule (I), (II) ou (III) : 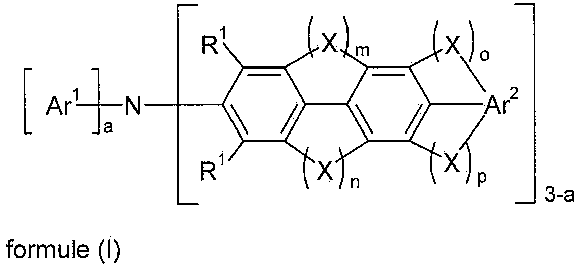 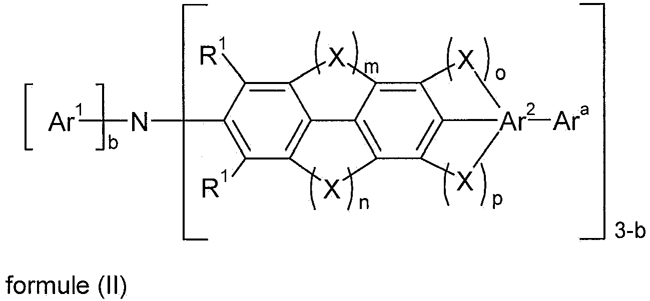 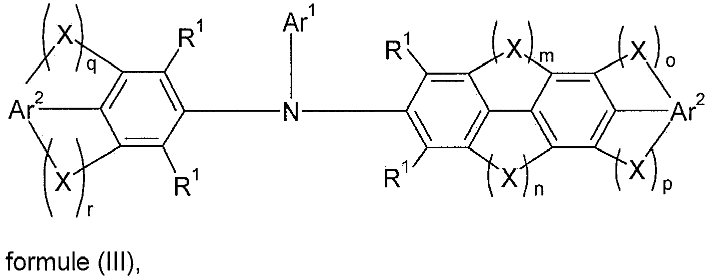 dans lesquelles :
Ar¹ est sélectionné pour chaque occurrence, de manière identique ou différente, parmi un système de cycle aromatique ou hétéroaromatique qui comporte de 5 à 30 atomes de cycle aromatique, lequel peut être substitué par un radical ou par plusieurs radicaux R¹ ;
Ar² est pour chaque occurrence, de manière identique ou différente, un groupe aryle ou hétéroaryle qui comporte de 10 à 30 atomes de cycle aromatique, lequel peut être substitué par un radical ou par plusieurs radicaux R¹ ;
Ar^{a} est pour chaque occurrence, de manière identique ou différente, un groupe aryle ou hétéroaryle qui comporte de 5 à 30 atomes de cycle aromatique, lequel peut être substitué par un radical ou par plusieurs radicaux R¹ ;
R¹ est pour chaque occurrence, de manière identique ou différente, H, D, F, Cl, Br, I, C(=O)R², CN, Si(R²)₃, N(R²)₂, NO₂, P(=O)(R²)₂, S(=O)R², S(=O)₂R², un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R² et où un ou plusieurs groupe(s) CH₂ dans les groupes qui ont été mentionnés ci-avant peut/peuvent être remplacé(s) par -R²C=CR²-, -C=C-, Si(R²)₂, C=O, C=S, C=NR², -C(=O)O-, -C(=O)NR²-, NR², P(=O)(R²), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte de 5 à 30 atomes de cycle aromatique, lequel peut dans chaque cas être substitué par un radical ou par plusieurs radicaux R², ou un groupe aryloxy ou hétéroaryloxy qui comporte de 5 à 30 atomes de cycle aromatique, lequel peut être substitué par un radical ou par plusieurs radicaux R², où deux radicaux R¹ qui sont liés au même groupe X peuvent former un cycle l'un avec l'autre ;
R² est pour chaque occurrence, de manière identique ou différente, H, D, F, Cl, Br, I, C(=O)R³, CN, Si(R³)₃, N(R³)₂, NO₂, P(=O)(R³)₂, S(=O)R³, S(=O)₂R³, un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être remplacés par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³ ;
R³ est pour chaque occurrence, de manière identique ou différente, H, D, F ou un radical aliphatique, aromatique ou hétéroaromatique qui comporte de 1 à 20 atome(s) de C, où, en outre, un ou plusieurs atome(s) de H peut/peuvent être remplacé(s) par D ou F ;
X est pour chaque occurrence, de manière identique ou différente, C(R¹)₂ ou Si(R¹)₂ ;
a est égal à 0 ou 1 ;
b est égal à 0, 1 ou 2 ;
m, n, o, p, q et r sont pour chaque occurrence, de manière identique ou différente, 0 ou 1 ; où, dans le cas dans lequel ils sont 0, un groupe R¹ est lié en lieu et place au niveau des positions pertinentes au niveau desquelles le groupe correspondant X est lié ;
où la somme de m et de n est égale à 1 ou 2, et la somme de o et de p est égale à 1 ou 2, et la somme de q et de r est égale à 1 ou 2.

2. Composé selon la revendication 1, **caractérisé en ce que** Ar¹ est sélectionné pour chaque occurrence, de manière identique ou différente, parmi phényle, naphtyle, anthracényle, phénanthrényle, pyrényle, triphénylényle, chrysényle, biphényle, terphényle, fluorényle, spirobifluorényle, carbazolyle, dibenzofuranyle, dibenzothiophényle ou silafluorényle, lequel peut dans chaque cas être substitué par un radical ou par plusieurs radicaux R¹.

3. Composé selon la revendication 1 ou 2, **caractérisé en ce que** Ar² est sélectionné parmi les groupes qui suivent des formules (Ar²-a) à (Ar²-h) :
| | |
|---|---|
| 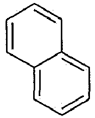 | 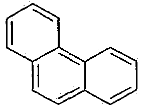 |
| formule Ar²-a) | formule Ar²-b) |
| 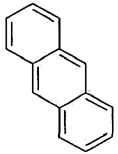 | 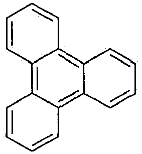 |
| formule (Ar²-c) | formule (Ar²-d) |
| 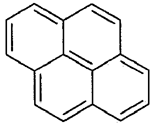 | 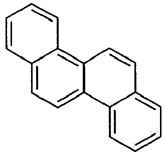 |
| formule (Ar²-e) | formule (Ar²-f) |
| 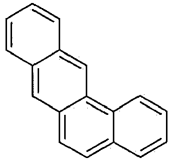 | 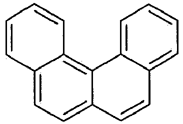 |
| formule (Ar²-g) | formule (Ar²-h) |
dans lesquelles les positions de liaison sur le radical de la formule peuvent être au niveau de n'importe quelles positions souhaitées et où les groupes peuvent être substitués par un radical ou par plusieurs radicaux R¹.

4. Composé selon une ou plusieurs des revendications 1 à 3, **caractérisé en ce que** Ar^{a} est un groupe aryle qui comporte de 6 à 18 atomes de cycle aromatique, lequel peut être substitué par un radical ou par plusieurs radicaux R¹.

5. Composé de la formule (III) selon une ou plusieurs des revendications 1 à 4, **caractérisé en ce que** Ar² est substitué par un groupe ou par plusieurs groupes R¹, lequel ou lesquels est/sont sélectionné(s) parmi un système de cycle aromatique ou hétéroaromatique qui comporte de 5 à 30 atomes de cycle aromatique, lequel peut être substitué par un radical ou par plusieurs radicaux R².

6. Composé selon une ou plusieurs des revendications 1 à 5, **caractérisé en ce que** R¹ est sélectionné pour chaque occurrence, de manière identique ou différente, parmi H, D, F, CN, Si(R²)₃, un groupe alkyle en chaîne droite qui comporte de 1 à 8 atome(s) de C ou un groupe alkyle ramifié ou cyclique qui comporte de 3 à 8 atomes de C, où les groupes alkyle peuvent dans chaque cas être substitués par un radical ou par plusieurs radicaux R² et où un ou plusieurs groupe(s) CH₂ dans les groupes alkyle peut/peuvent être remplacé(s) par -C≡C-, -R²C=CR²-, Si(R²)₂, C=O ou -O-, ou un groupe aryle ou hétéroaryle qui comporte de 6 à 16 atomes de cycle aromatique, lequel peut dans chaque cas être substitué par un radical ou par plusieurs radicaux R².

7. Composé selon une ou plusieurs des revendications 1 à 6, **caractérisé en ce que** a est égal à 1.

8. Composé selon une ou plusieurs des revendications 1 à 7, **caractérisé en ce que** b est égal à 2.

9. Composé selon une ou plusieurs des revendications 1 à 8, **caractérisé en ce que** la somme de q et de r est égale à 1.

10. Oligomères, polymères ou dendrimères contenant un ou plusieurs composé(s) selon une ou plusieurs des revendications 1 à 9, où la/les liaison(s) sur le polymère, l'oligomère ou le dendrimère peut/peuvent être localisée(s) au niveau de n'importe quelles positions souhaitées dans la formule (I), (II) ou (III) qui sont substituées par R¹ ou R².

11. Formulation comprenant au moins un composé selon une ou plusieurs des revendications 1 à 9 ou au moins un polymère, un oligomère ou un dendrimère selon la revendication 10 et au moins un solvant.

12. Dispositif électronique, sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED), **caractérisé en ce qu'**il contient au moins un composé selon une ou plusieurs des revendications 1 à 9 ou au moins un polymère, un oligomère ou un dendrimère selon la revendication 10.

13. Dispositif électronique selon la revendication 12, sélectionné parmi les dispositifs électroluminescents, **caractérisé en ce que** le composé selon une ou plusieurs des revendications 1 à 9 ou le polymère, l'oligomère ou le dendrimère selon la revendication 10 est utilisé en tant que matériau d'émission dans une couche d'émission.

14. Procédé pour la préparation d'un composé selon une ou plusieurs des revendications 1 à 9, **caractérisé en ce qu'**un ou plusieurs processus de couplage d'organométallique(s) est/sont utilisé(s).
